# EUROPEAN PATENT SPECIFICATION

(11) **EP 4 270 067 B1**
(45) Date of publication and mention of the grant of the patent: **03.12.2025**
(21) Application number: 21911102.8
(22) Date of filing: 24.12.2021
(51) Int. Cl.: C09K 9/02, G02C 7/10, C07D 311/78, G02B 1/04, G02B 5/23, C07D 311/94, C07D 407/04, C07D 409/04, C07D 497/04, C07F 7/04

(54) **PHOTOCHROMIC COMPOSITION, PHOTOCHROMIC ARTICLE, AND GLASSES**
PHOTOCHROME ZUSAMMENSETZUNG, PHOTOCHROMER ARTIKEL UND GLÄSER
COMPOSITION PHOTOCHROMIQUE, ARTICLE PHOTOCHROMIQUE ET VERRES

(30) Priority: 24.12.2020 JP 2020215158; 24.12.2020 JP 2020215159; 08.02.2021 JP 2021018610
(43) Date of publication of application: 01.11.2023
(73) Proprietor: HOYA LENS THAILAND LTD., Pathumthani 12130 (TH)
(72) Inventor: KAWAKAMI Hironori, Tokyo 160-8347 (JP); KOBAYASHI Kei, Tokyo 160-8347 (JP); MATSUE Aoi, Tokyo 160-8347 (JP)
(74) Representative: Hoffmann Eitle
(86) International application number: PCT/JP2021/048398
(87) International publication number: WO 2022/138965

(56) References cited:
- EP-A1- 2 479 171
- WO-A1-2009/136668
- WO-A1-2011/053615
- WO-A1-2013/078086
- WO-A1-2014/151543
- JP-A- 2014 506 245
- JP-A- 2014 507 385
- JP-A- 2014 510 718
- US-A1- 2012 145 973
- US-A1- 2016 279 886

## Description

### [Technical Field]

The present invention relates to a photochromic composition, a photochromic article, and spectacles.

### [Background Art]

A photochromic compound is a compound having properties (photochromic properties) of coloring under irradiation with light in a wavelength range having photoresponsivity and fading under non-irradiation. For example, PTL 1 and [PTL 2] disclose a naphthopyran-based compound having photochromic properties.

### [Citation List]

### [Patent Literature]

[PTL 1] WO 2000/15631
[PTL 2] EP 2 479 171 A1

### [Summary of Invention]

### [Technical Problem]

Examples of methods of imparting photochromic properties to optical articles such as spectacle lenses include a method of incorporating a photochromic compound into a substrate, and a method of forming a layer containing a photochromic compound. Examples of performance desired for such optical articles to which photochromic properties have been imparted include a high coloring density when coloring in the visible range (wavelength: 380 to 780 nm), and exhibition of a fast fading speed after coloring by irradiation with light.

An object of one aspect of the present invention is to provide a photochromic article having a high coloring density when coloring in the visible range and a high fading speed.

### [Solution to Problem]

One aspect of the present invention relates to a photochromic article containing: one or more compounds represented by General Formula A; and one or more compounds represented by General Formula B.

Another aspect of the present invention relates to a photochromic composition containing: one or more compounds represented by General Formula A; and one or more compounds represented by General Formula B.

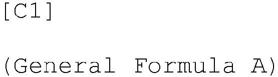

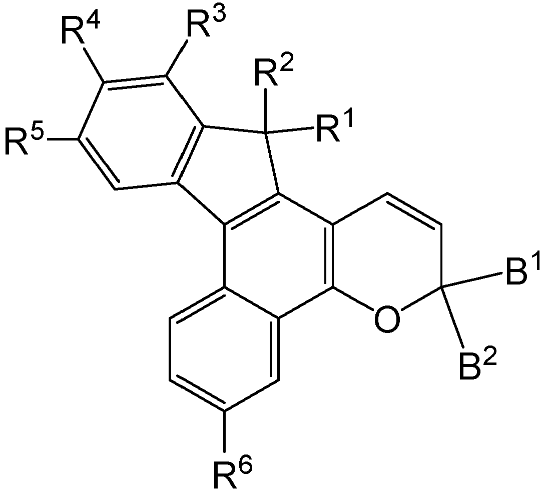

In General Formula A, R¹, R², B¹, and B² each independently represent a hydrogen atom or a substituent, and R³ to R⁶ each independently represent a hydrogen atom or an electron-withdrawing group, provided that one or more of R³ to R⁶ represent an electron-withdrawing group.

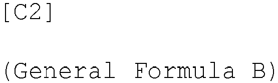

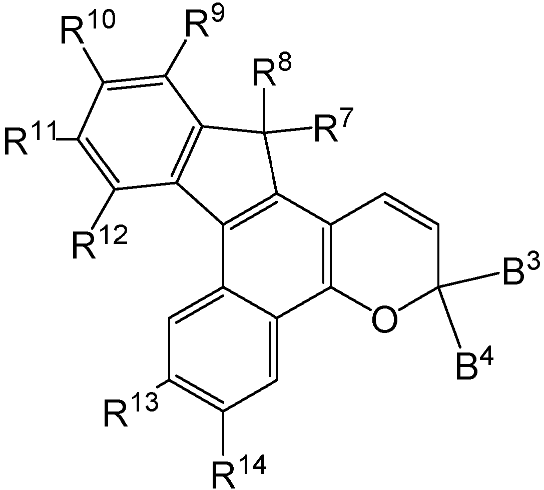

In General Formula B, R⁷ to R¹², B³, and B⁴ each independently represent a hydrogen atom or a substituent, and R¹³ and R¹⁴ each independently represent a hydrogen atom or an electron-donating group, provided that one or more of R¹³ and R¹⁴ represent an electron-donating group.

As a result of intensive studies by the inventors of the present invention, they newly found that by combining the compound represented by General Formula A and the compound represented by General Formula B, a photochromic article that can color at a high density in the visible range can be provided. This is presumed to be because the compound represented by General Formula A and the compound represented by General Formula B have different absorption peak positions in the visible range, and therefore, by combining these compounds, coloring at a high density in a wide wavelength range becomes possible.

Furthermore, it was newly found that by combining the compound represented by General Formula A and the compound represented by General Formula B, a photochromic article capable of exhibiting a fast fading speed can be provided.

### [Advantageous Effects of Invention]

According to one aspect of the present invention, a photochromic article having a high coloring density when coloring in the visible range and a high fading speed can be provided.

### [Description of Embodiments]

As an example, a photochromic compound undergoes structural conversion into a colored product via an excited state upon irradiation with light such as sunlight. The structure after structural conversion via irradiation with light can be called a "colored product". In contrast, the structure before irradiation with light can be called a "colorless product". However, the term "colorless" in the colorless product is not limited to being completely colorless and also includes a case in which a color is lighter than that of the colored product. Each of the structure of General Formula A and the structure of General Formula B is the structure of the colorless product.

In the present invention and the present specification, the term "photochromic article" refers to an article containing a photochromic compound. A photochromic article according to one aspect of the present invention contains, as photochromic compounds, one or more compounds represented by General Formula A and one or more compounds represented by General Formula B. The photochromic compound can be contained in a substrate of the photochromic article and/or can be contained in a photochromic layer in the photochromic article having a substrate and the photochromic layer. The "photochromic layer" is a layer containing a photochromic compound.

In the present invention and the present specification, the term "photochromic composition" refers to a composition containing the photochromic compound. The photochromic composition according to one aspect of the present invention contains, as photochromic compounds, one or more compounds represented by General Formula A and one or more compounds represented by General Formula B, and can be used for the production of the photochromic article according to one aspect of the present invention.

In the present invention and the present specification, various substituents, that is, substituents that can be represented by any of R¹, R², B¹, and B² in General Formula A and R⁷ to R¹², B³, and B⁴ in General Formula B and substituents when each group described later has a substituent may each independently be the following substituent:
a substituent R^{m} selected from the group consisting of a linear or branched alkyl group having 1 to 18 carbon atoms such as a hydroxy group, a methyl group, an ethyl group, a propyl group, a butyl group, a pentyl group, and a hexyl group; a cycloaliphatic alkyl group of a single-ring type or a multi-ring type such as a bicyclic ring which has 5 to 18 carbon atoms such as a cyclopentyl group and a cyclohexyl group; a linear or branched alkoxy group having 1 to 24 constituent atoms such as a methoxy group, an ethoxy group, and a butoxy group; a non-aromatic cyclic substituent having 1 to 24 constituent atoms; a linear or branched perfluoroalkyl group having 1 to 18 carbon atoms such as a trifluoromethyl group; a linear or branched perfluoroalkoxy group such as a trifluoromethoxy group; a linear or branched alkylsulfide group having 1 to 24 constituent atoms such as a methylsulfide group, an ethylsulfide group, and a butylsulfide group; an aryl group such as a phenyl group, a naphthyl group, an anthracenyl group, a fluoranthenyl group, a phenanthryl group, a pyranyl group, a perylenyl group, a styryl group, and a fluorenyl group; an aryloxy group such as a phenyloxy group; an arylsulfide group such as a phenylsulfide group; a heteroaryl group such as a pyridyl group, a furanyl group, a thienyl group, a pyrrolyl group, a benzofuranyl group, a benzothiophenyl group, an indolyl group, a dibenzofuranyl group, a dibenzothiophenyl group, a carbazolyl group, a diazolyl group, a triazolyl group, a quinolinyl group, a phenothiazinyl group, a phenoxazinyl group, a phenazinyl group, a thianthryl group, and an acridinyl group; an amino group (-NH₂); a monoalkylamino group such as a monomethylamino group; a dialkylamino group such as a dimethylamino group; a monoarylamino group such as a monophenylamino group; a diarylamino group such as a diphenylamino group; a cyclic amino group such as a piperidino group, a morpholino group, a thiomorpholino group, a tetrahydroquinolino group, and a tetrahydroisoquinolino group; an ethynyl group; a mercapto group; a silyl group; a sulfonic acid group; an alkylsulfonyl group; a formyl group; a carboxy group; a cyano group; and a halogen atom such as a fluorine atom, a chlorine atom, a bromine atom, and an iodine atom, or
a substituent in which R^{m} is further substituted with one or more same or different R^{m'} s.

Examples of the above-mentioned substituent in which R^{m} is further substituted with one or more same or different R^{m'} s include a structure in which a carbon atom at the terminal of an alkoxy group is further substituted with an alkoxy group, and a carbon atom at the terminal of this alkoxy group is further substituted with an alkoxy group. In addition, other examples of the above-mentioned substituent in which R^{m} is further substituted with one or more same or different R^{m'} s include a structure in which two or more positions of the five substitutable positions of a phenyl group are substituted with the same or different R^{m'} s. However, the above-mentioned substituent is not limited to such examples.

In the present invention and the present specification, the terms "the number of carbon atoms" and "the number of constituent atoms" refer to the numbers including the number of carbon atoms or the number of atoms of a substituent when referring to a group having a substituent.

In addition, in the present invention and the present specification, various substituents, that is, substituents that can be represented by any of R¹, R², B¹, and B² in General Formula A and R⁷ to R¹², B³, and B⁴ in General Formula B and substituents when each group described later has a substituent may each independently be a solubilizing group. In the present invention and the present specification, the term "solubilizing group" refers to a substituent that can contribute to enhancing compatibility with an arbitrary liquid or a specific liquid. As the solubilizing group, a substituent is suitable, the substituent capable of contributing to promoting thermal motion of the molecule of a compound by having the following substituent: an alkyl group having a linear, branched, or cyclic structure and having 4 to 50 carbon atoms; a linear, branched, or cyclic alkoxy group having 4 to 50 constituent atoms; a linear, branched, or cyclic silyl group having 4 to 50 constituent atoms; a substituent in which a part of the above-mentioned group has been substituted with a silicon atom, a sulfur atom, a nitrogen atom, a phosphorus atom, or the like; a substituent in which two or more of the above-mentioned groups have been combined; and the like. A compound having the solubilizing group as a substituent can be made to have a molecular association state close to that in a liquid by inhibiting the distance between solute molecules from becoming closer to prevent the solidification of a solute, or by lowering the melting point and/or glass transition temperature of a solute. Thereby, the solubilizing group can liquefy a solute or can increase the solubility of a compound having this substituent in a liquid. In one aspect, as the solubilizing group, a n-butyl group, a n-pentyl group, a n-hexyl group, and a n-octyl group which are linear alkyl groups; a tert-butyl group which is a branched alkyl group; and a cyclopentyl group and a cyclohexyl group which are cyclic alkyl groups are preferable.

The above-mentioned substituent is preferably a substituent selected from the group consisting of a methoxy group, an ethoxy group, a phenoxy group, a methylsulfide group, an ethylsulfide group, a phenylsulfide group, a trifluoromethyl group, a phenyl group, a naphthyl group, a dibenzofuranyl group, a dibenzothiophenyl group, a carbazolyl group, a phenothiazinyl group, a phenoxazinyl group, a phenazinyl group, an acridinyl group, a dimethylamino group, a diphenylamino group, a piperidino group, a morpholino group, a thiomorpholino group cyano group, and a solubilizing group, and is more preferably a substituent selected from the group consisting of a methoxy group, a phenoxy group, a methylsulfide group, a phenylsulfide group, a trifluoromethyl group, a phenyl group, a dimethylamino group, a diphenylamino group, a piperidino group, a morpholino group, a thiomorpholino group cyano group, and a solubilizing group.

In the present invention and the present specification, the term "electron-withdrawing group" refers to a substituent that more easily attracts electrons from a bonding atom side, as compared to a hydrogen atom. The electron-withdrawing group can attract electrons as a result of substituent effects such as an inductive effect and a mesomeric effect (or resonance effect). Specific examples of electron-withdrawing groups include a halogen atom (fluorine atom: -F, chlorine atom: -Cl, bromine atom: -Br, iodine atom: -I), a trifluoromethyl group: -CF₃, a nitro group: -NO₂, a cyano group: -CN, a formyl group: -CHO, an acyl group: -COR (where R is a substituent), an alkoxycarbonyl group: -COOR, a carboxy group: -COOH, a substituted sulfonyl group: -SO₂R (where R is a substituent), and a sulfo group: -SO₃H. Examples of suitable electron-withdrawing groups include a fluorine atom that is an electron-withdrawing group having a high electronegativity, and an electron-withdrawing group in which a substituent constant σₚ for para-positions based on the Hammett equation is a positive value.

In the present invention and the present specification, the term "electron-donating group" refers to a substituent that more easily donates electrons to a bonding atom side, as compared to a hydrogen atom. The electron-donating group can be a substituent that easily donates electrons due to the sum of the inductive effect, the mesomeric effect (or resonance effect), and the like. Specific examples of electron-donating groups include a hydroxy group: -OH, a thiol group: -SH, an alkoxy group: -OR (where R is an alkyl group), an alkylsulfide group: -SR (where R is an alkyl group), an arylsulfide group, an acetyl group: -OCOCH₃, an amino group: -NH₂, an alkylamide group: -NHCOCH₃, a dialkylamino group: -N(R)₂ (where two R's are the same or different alkyl groups or aryl groups, and R's may be bonded to each other to form a ring structure), and a methyl group. Examples of suitable electron-donating groups include an electron-donating group in which a substituent constant σₚ for para-positions based on the Hammett equation is a negative value.

Specific examples of substituent constants σₚ for para-positions based on the Hammett equation (source: Organic Chemistry for Graduate School (Volume 1) (1988) by Hiizu IWAMURA, Ryoji NOYORI, Takeshi NAKAI, and Isao KITAGAWA) are described below.
-N(CH₃)₂: -0.83
-OCH3: -0.27
-t-C₄H₉: -0.20
-CH₃: -0.17
-C₂H₅: -0.15
-C₆H₅: -0.01
(-H: 0)
-F: +0.06
-Cl: +0.27
-Br: +0.23
-CO₂C2H₅: +0.45
-CF₃: +0.54
-CN : +0.66
-SO₂CH₃: +0.72
-NO₂: +0.78

Hereinbelow, the compound represented by General Formula A and the compound represented by General Formula B will be described in more detail.

### <Compound Represented by General Formula A>

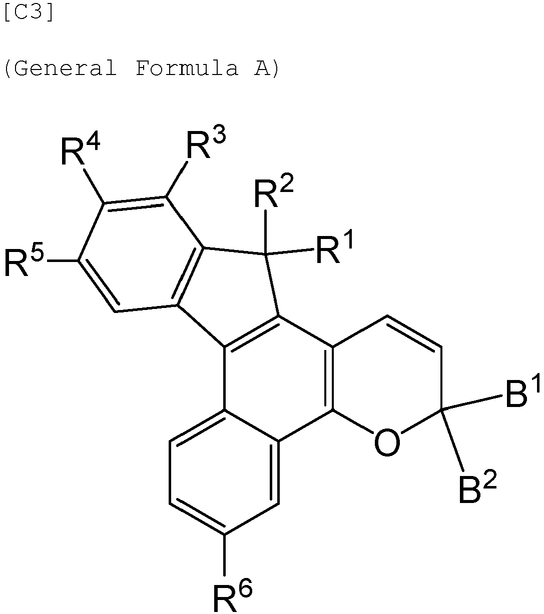

In General Formula A, R¹, R², B¹, and B² each independently represent a hydrogen atom or a substituent.

Preferably, R¹ and R² each independently represent a substituted or unsubstituted alkyl group having 1 to 20 carbon atoms, and more preferably, R¹ and R² each independently represent a methyl group, an ethyl group, a propyl group, a butyl group, a pentyl group, or a hexyl group. Further preferably, R¹ and R² each independently represent a methyl group or an ethyl group, and still further preferably, both R¹ and R² represent a methyl group or both R¹ and R² represent an ethyl group.

Preferably, B¹ and B² each independently represent a substituted or unsubstituted phenyl group. When the phenyl group has multiple substituents, two or more of these substituents may be bonded to form a ring. Specific examples of rings to be formed include rings included in exemplary compounds to be described later. The substitution position of a substituent in a substituted phenyl group is preferably a position that is the para-position with respect to the carbon atom to which B¹ and B² are bonded. Specific examples of substituents of the substituted phenyl group include substituents, which are included in exemplary compounds to be described later, such as a morpholino group, a piperidino group, a halogen atom, an alkoxy group, and the following substituents. In the present invention and the present specification, the symbol "*" relating to a partial structure of a compound indicates a bonding position with the atom to which such a partial structure is bonded.

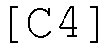

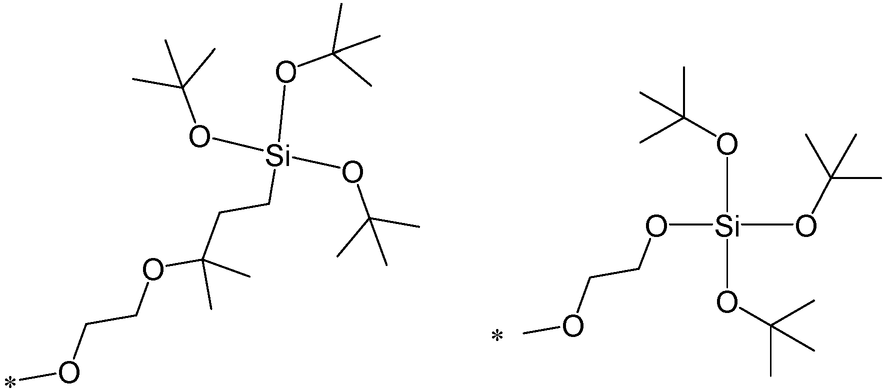

In General Formula A, R³ to R⁶ each independently represent a hydrogen atom or an electron-withdrawing group. However, one or more of R³ to R⁶ represent an electron-withdrawing group. As the electron-withdrawing group, a halogen atom, a perfluoroalkyl group having 1 to 6 carbon atoms, a perfluorophenyl group, a perfluoroalkylphenyl group, or a cyano group is preferable. As the halogen atom, a fluorine atom is preferable. As the perfluoroalkyl group having 1 to 6 carbon atoms, a trifluoromethyl group is preferable.

In one aspect, the compound represented by General Formula A can be the following compounds.

A compound in which only R⁴ among R³ to R⁶ is an electron-withdrawing group, and R³, R⁵, and R⁶ are hydrogen atoms.

A compound in which R⁴ and R⁶ are the same or different electron-withdrawing groups among R³ to R⁶, and R³ and R⁵ are hydrogen atoms.

A compound in which R³ and R⁵ are the same or different electron-withdrawing groups among R³ to R⁶, and R⁴ and R⁶ are hydrogen atoms.

Examples of the compounds represented by General Formula A include the following compounds. However, the present invention is not limited to the compounds exemplified below.

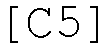

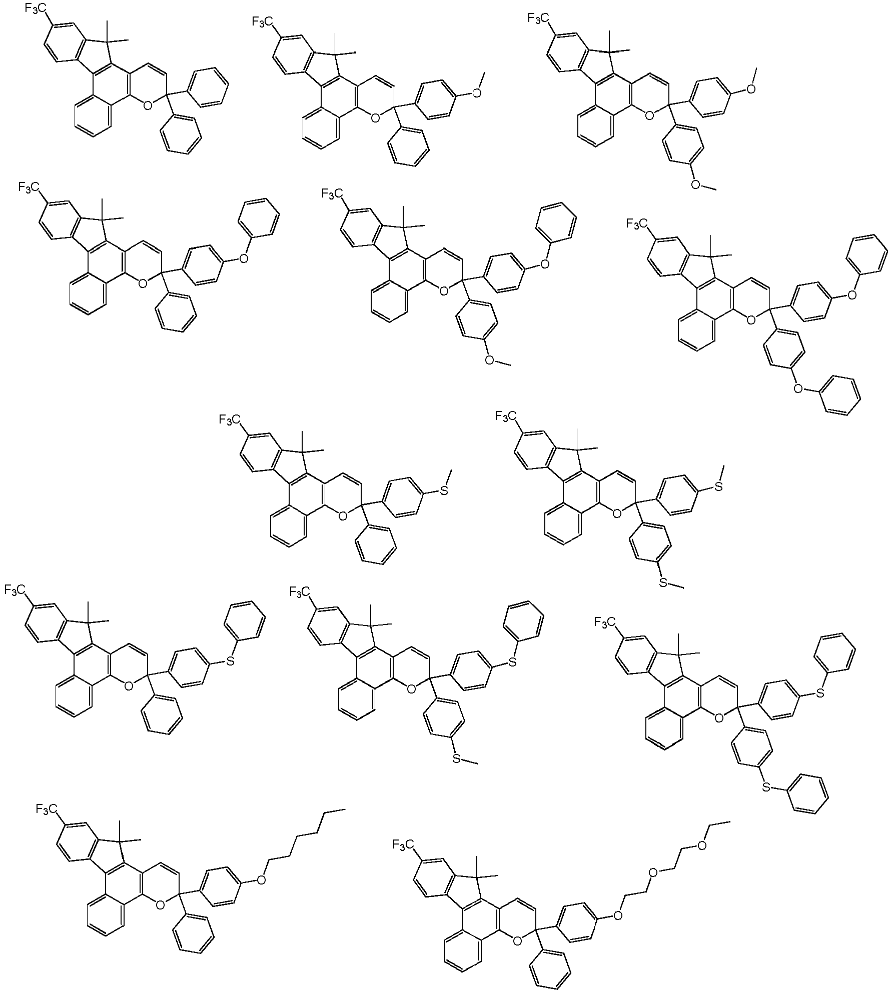

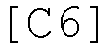

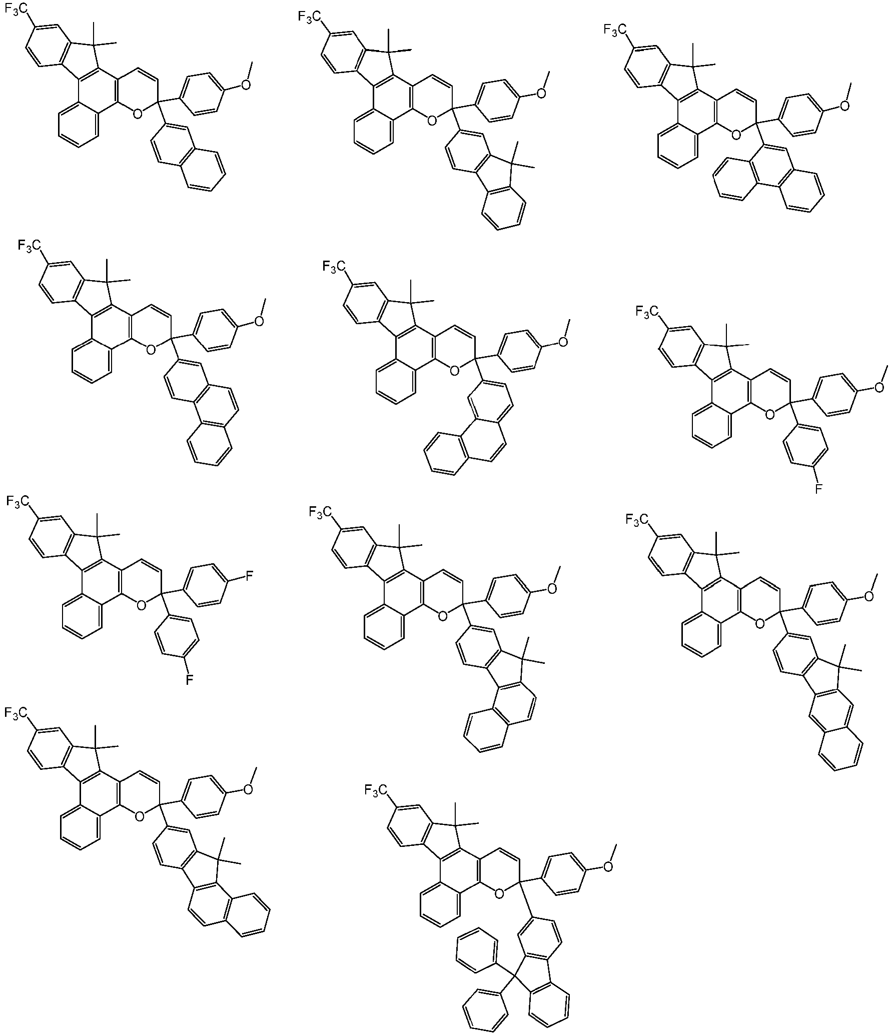

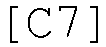

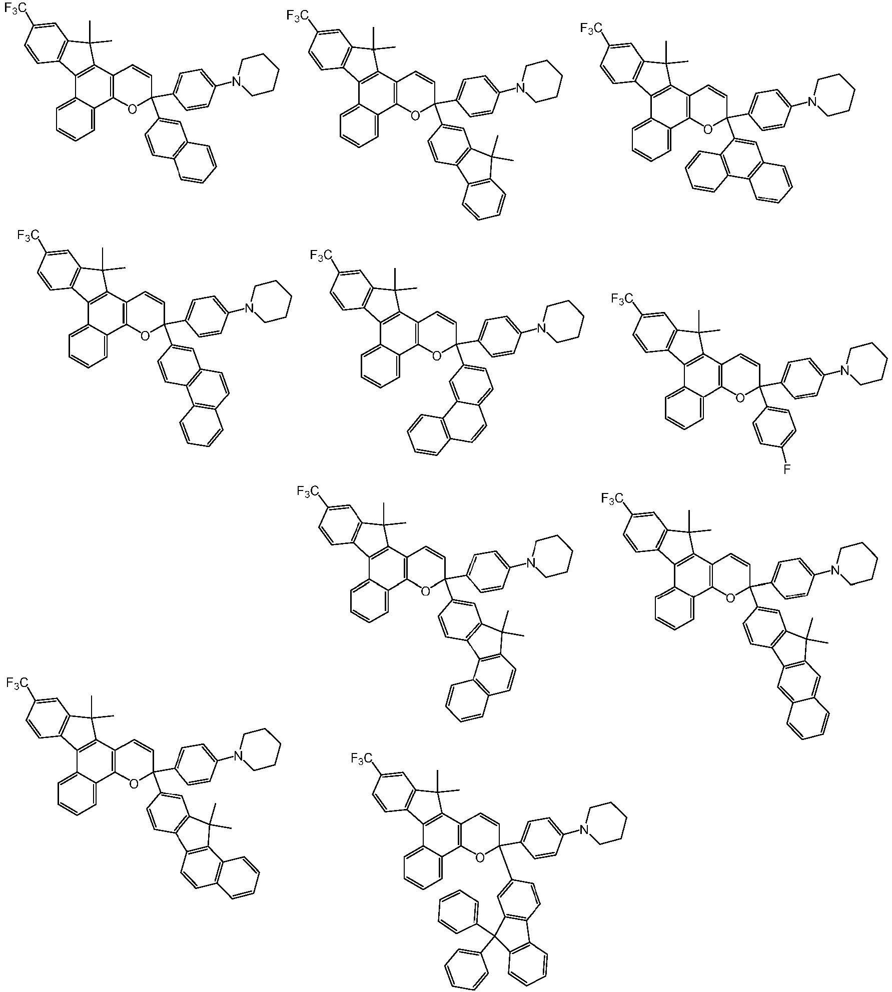

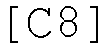

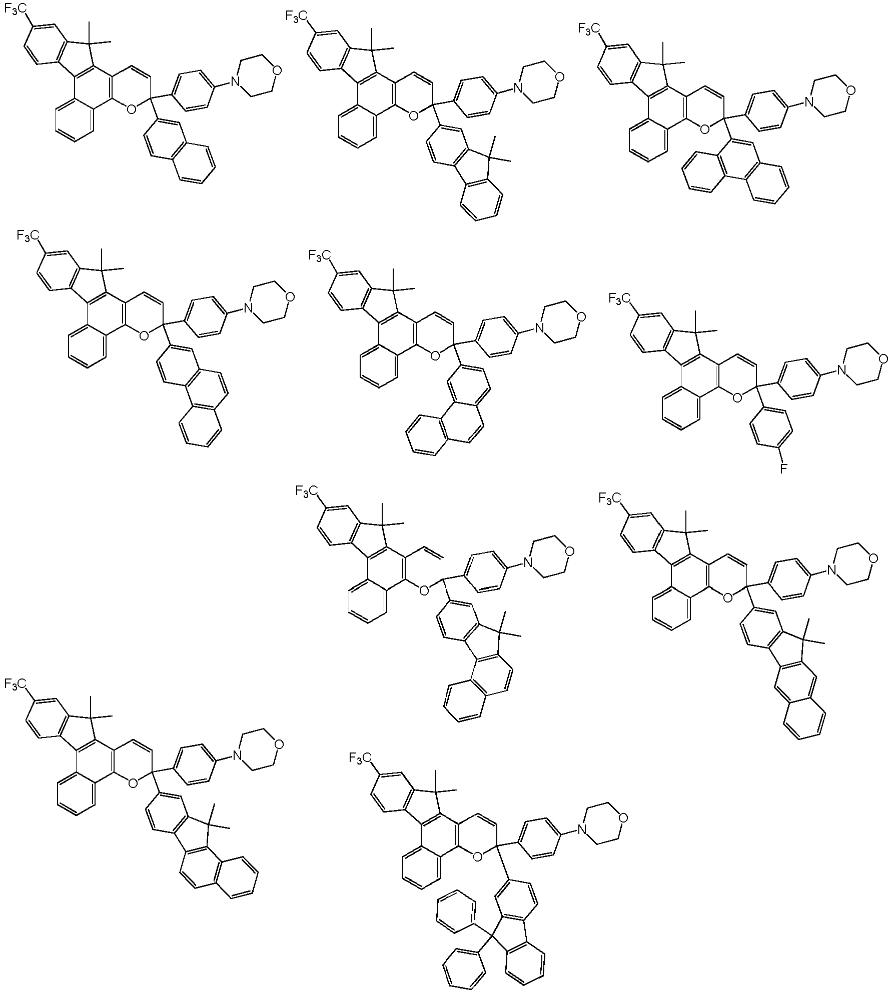

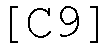

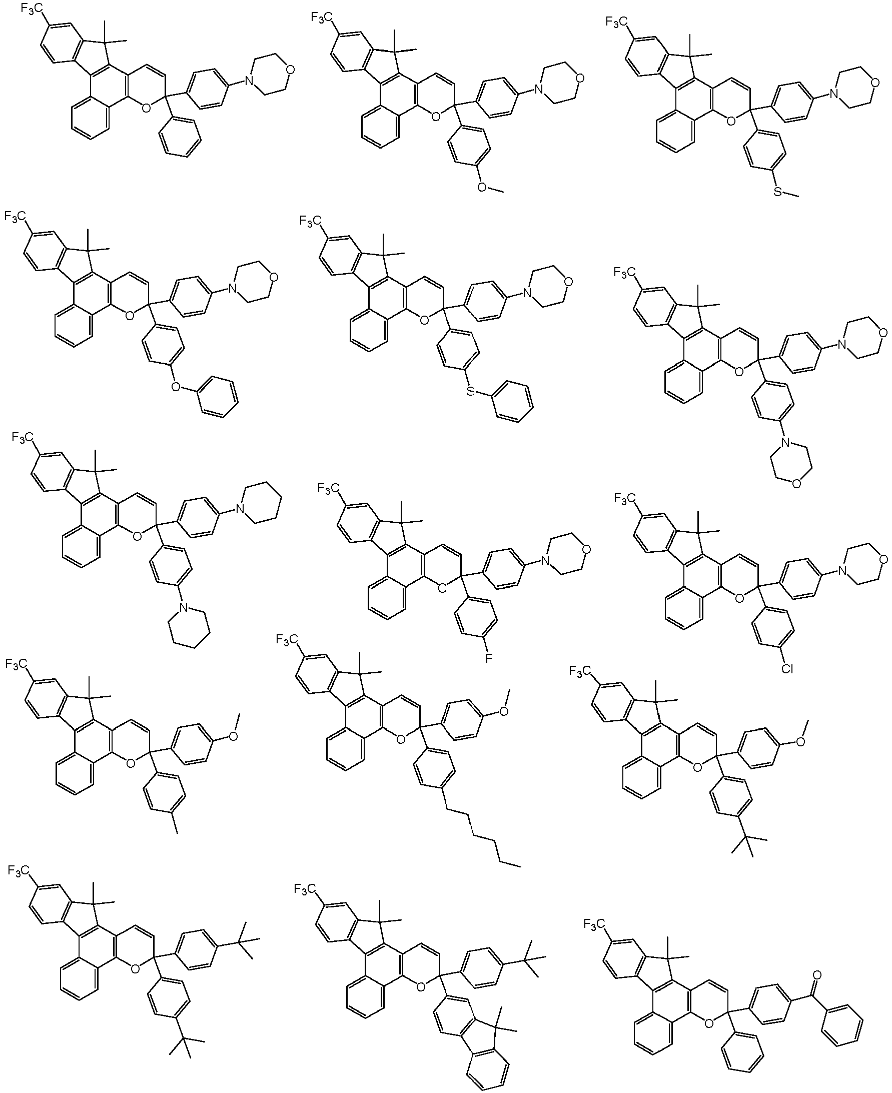

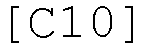

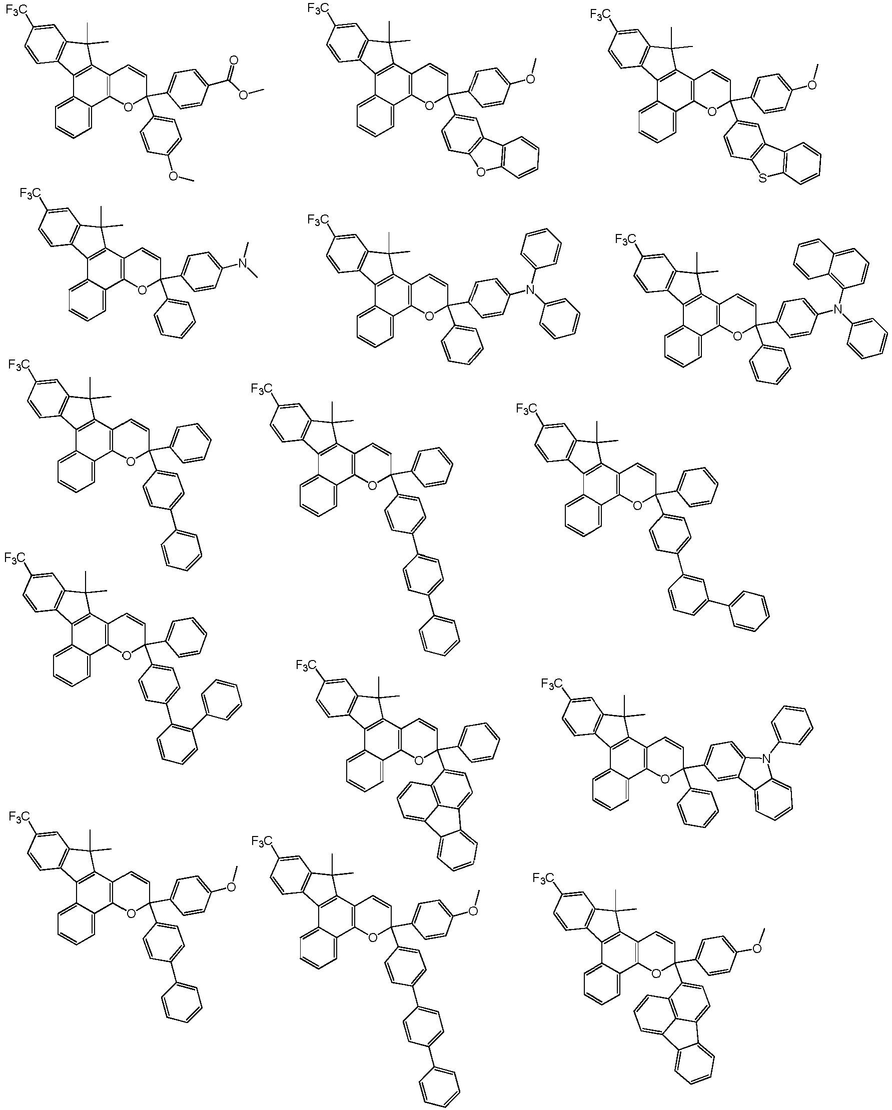

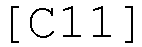

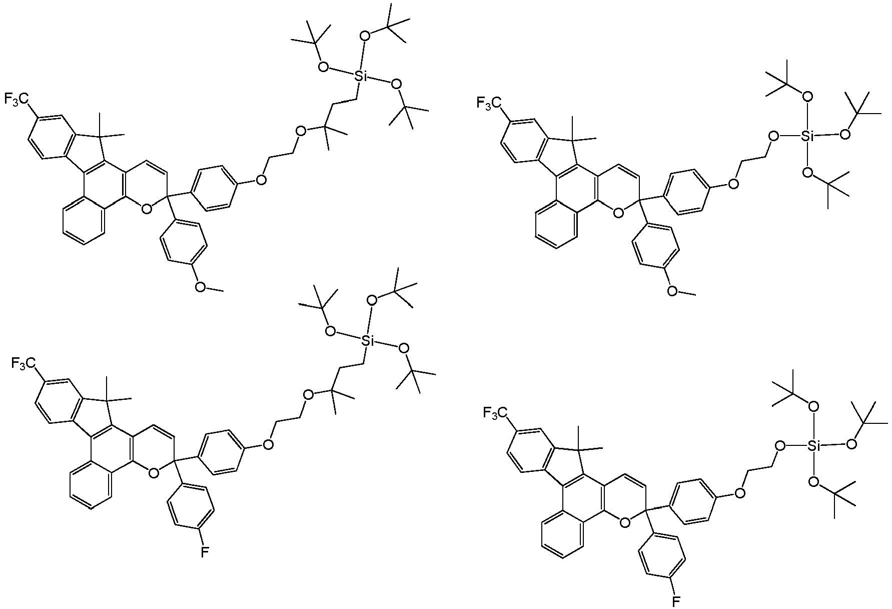

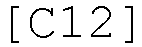

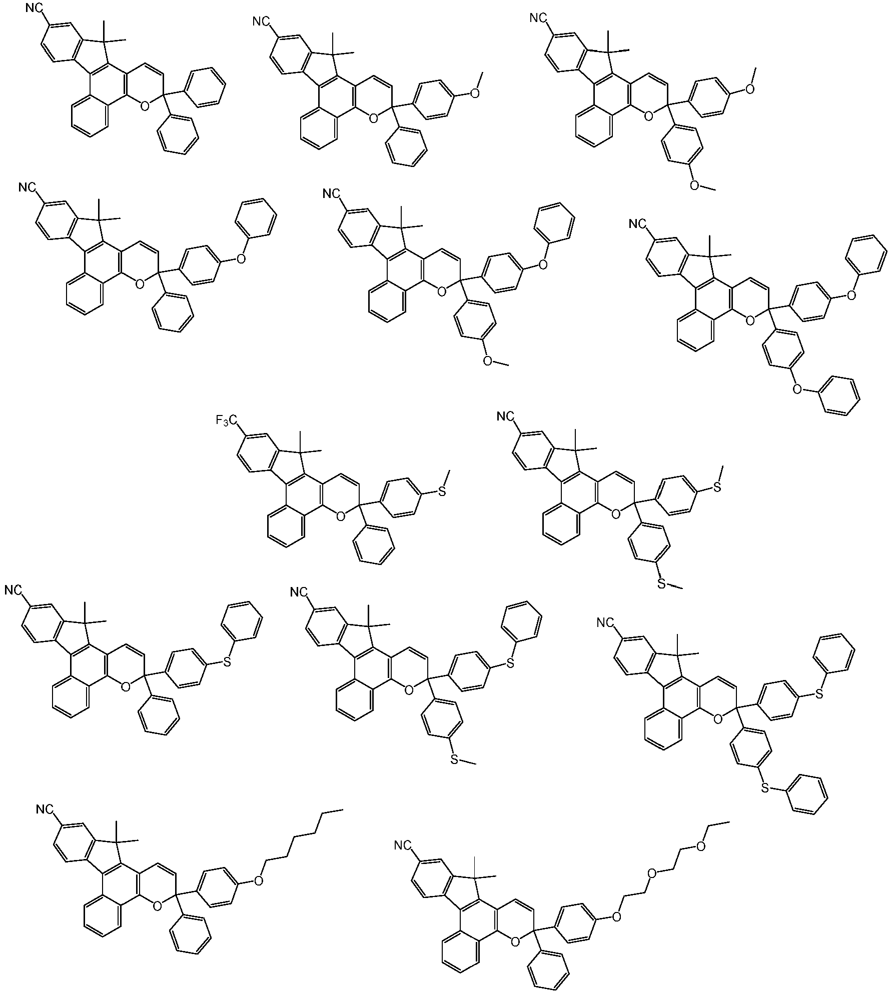

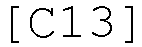

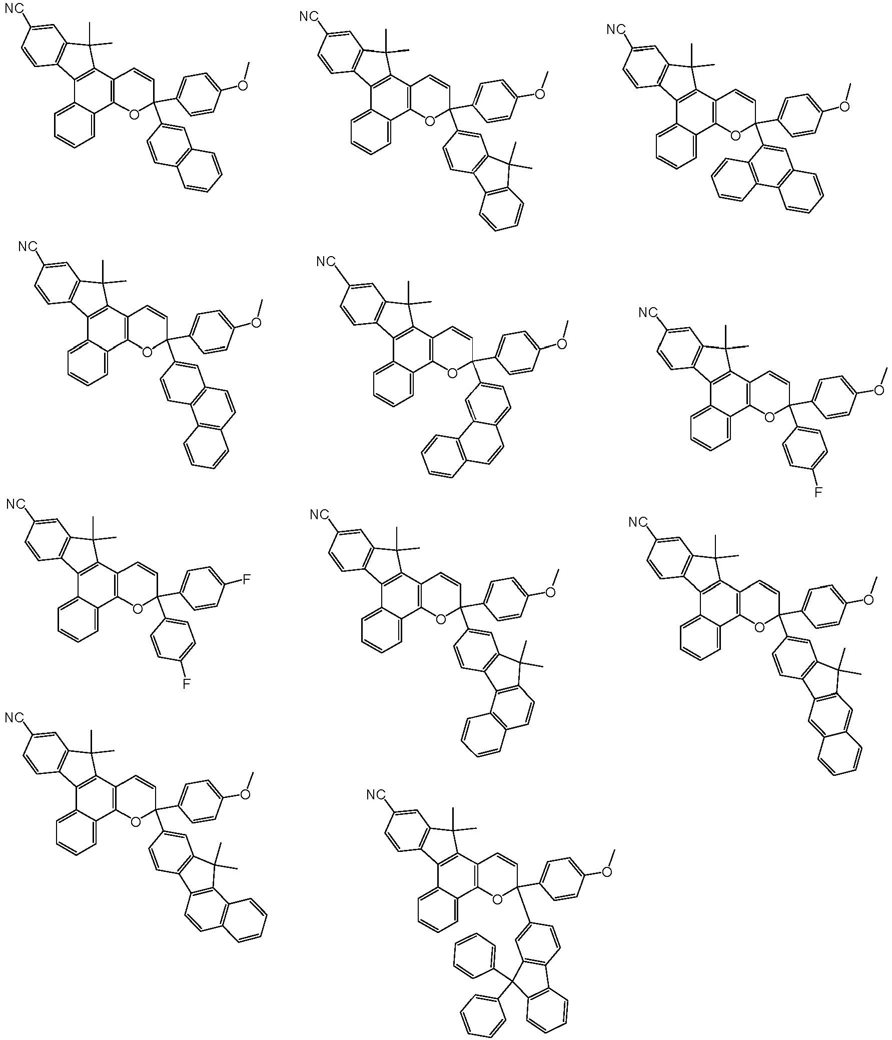

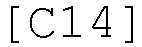

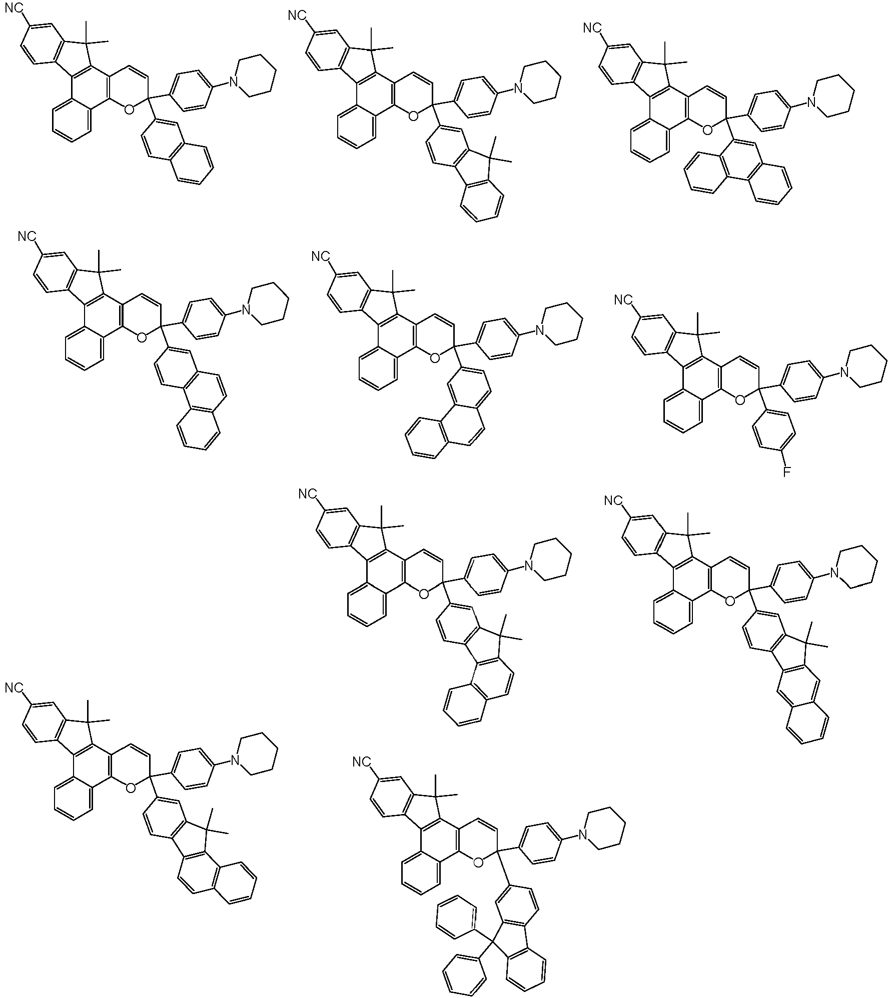

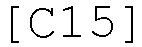

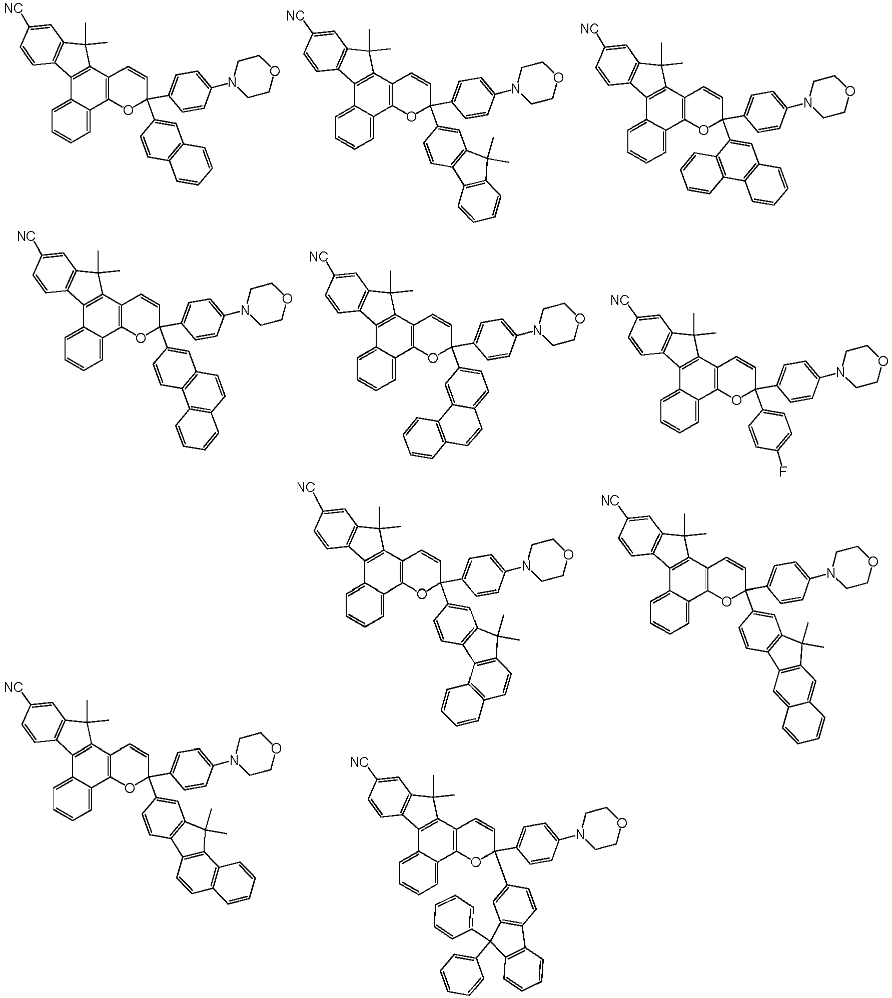

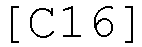

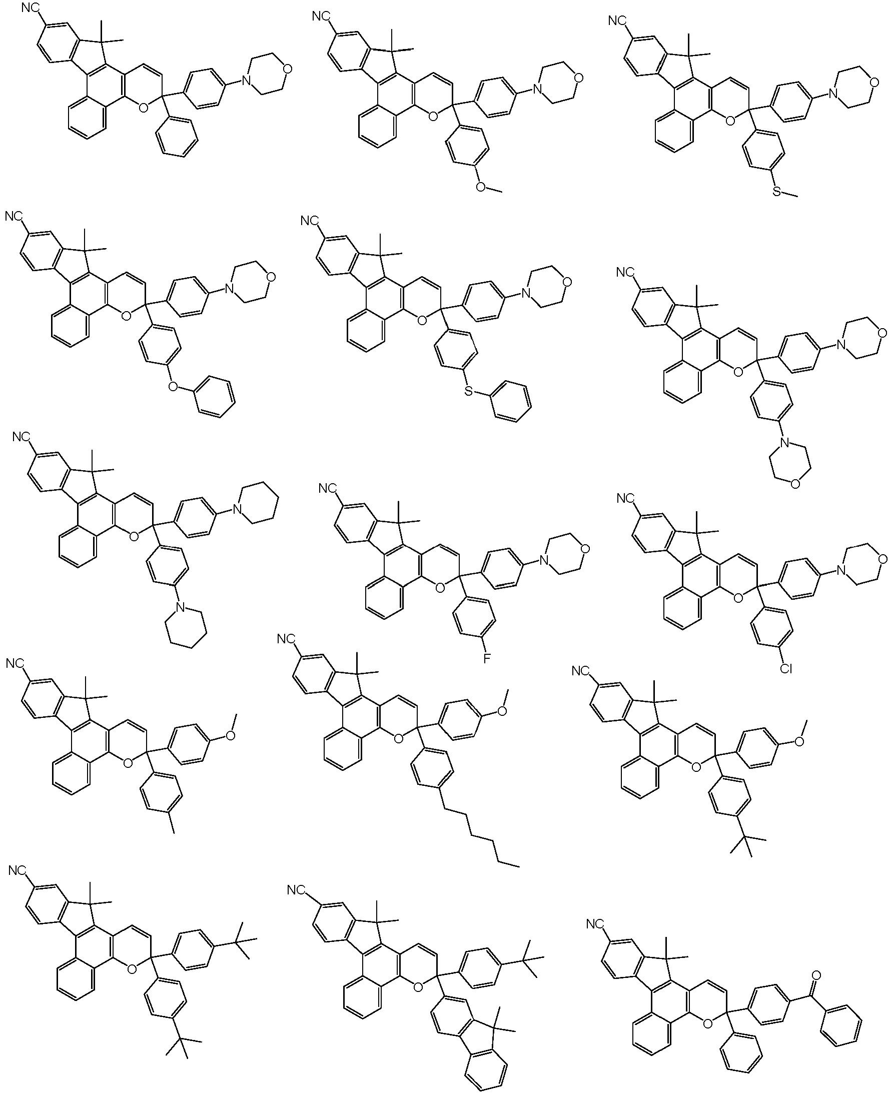

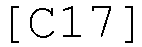

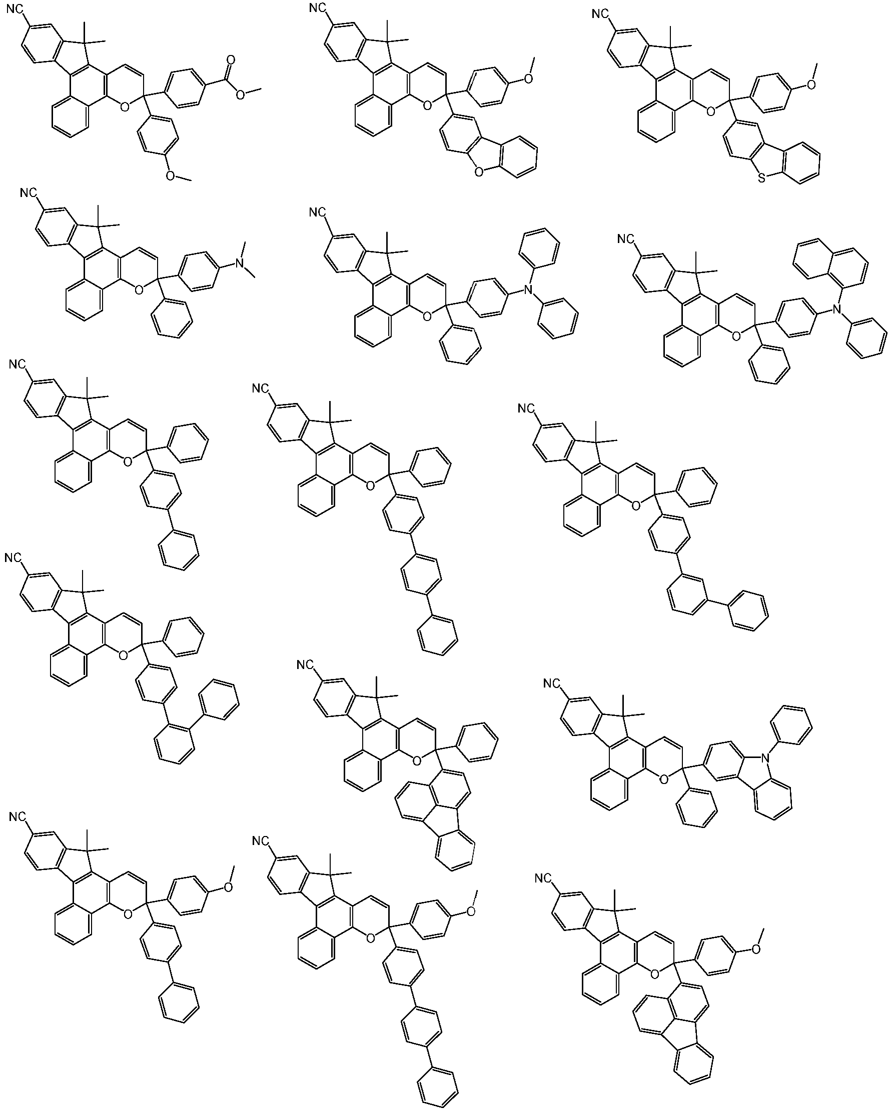

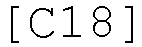

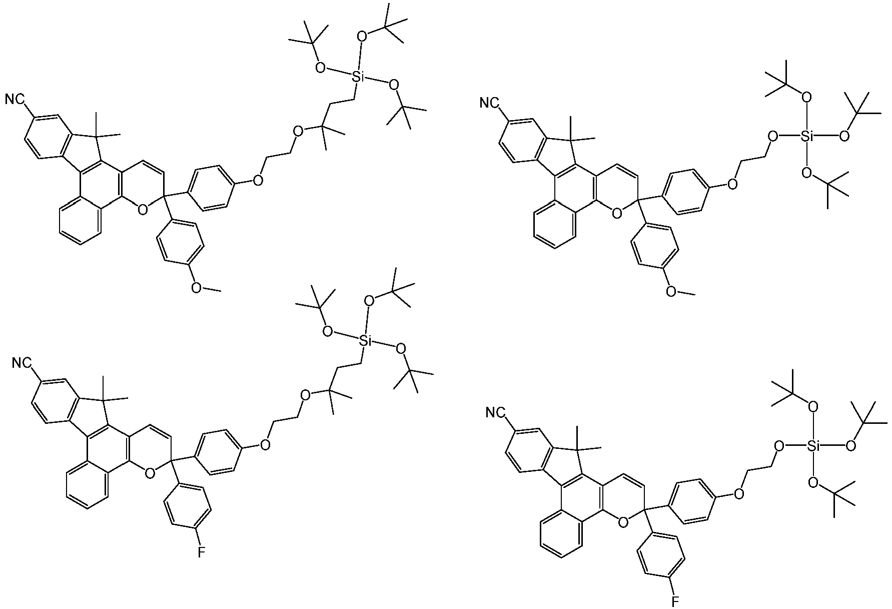

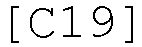

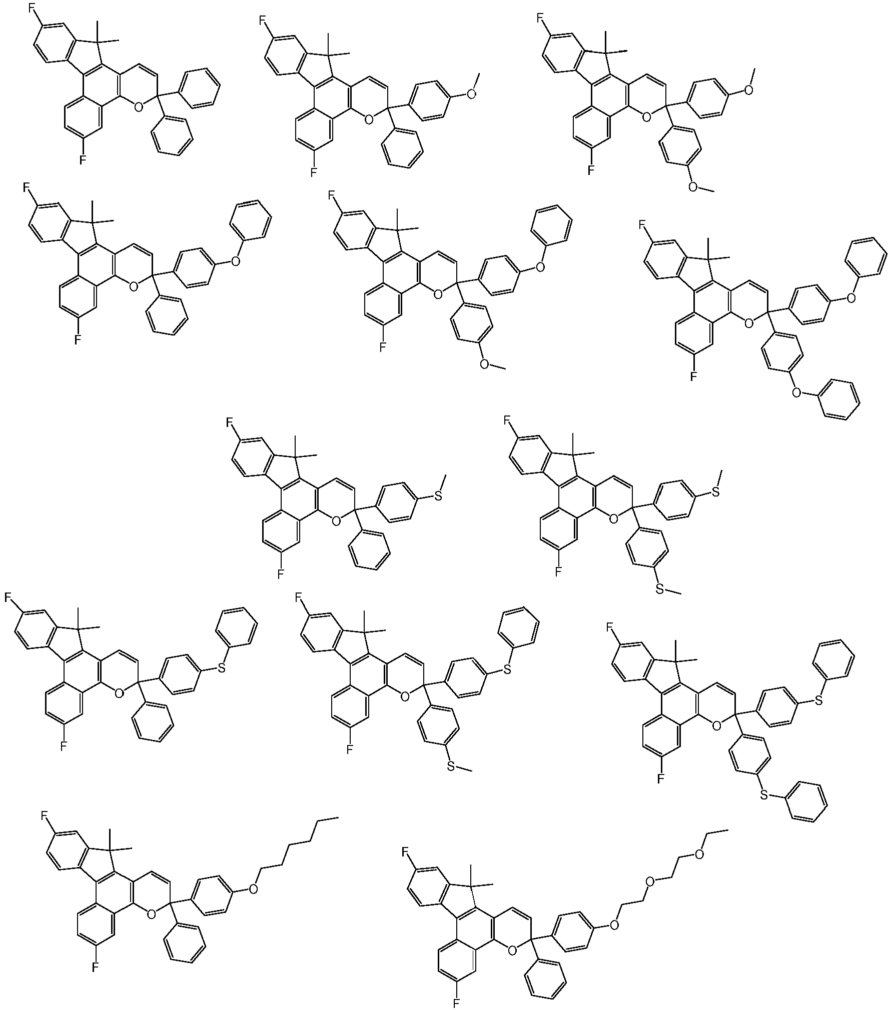

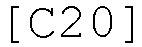

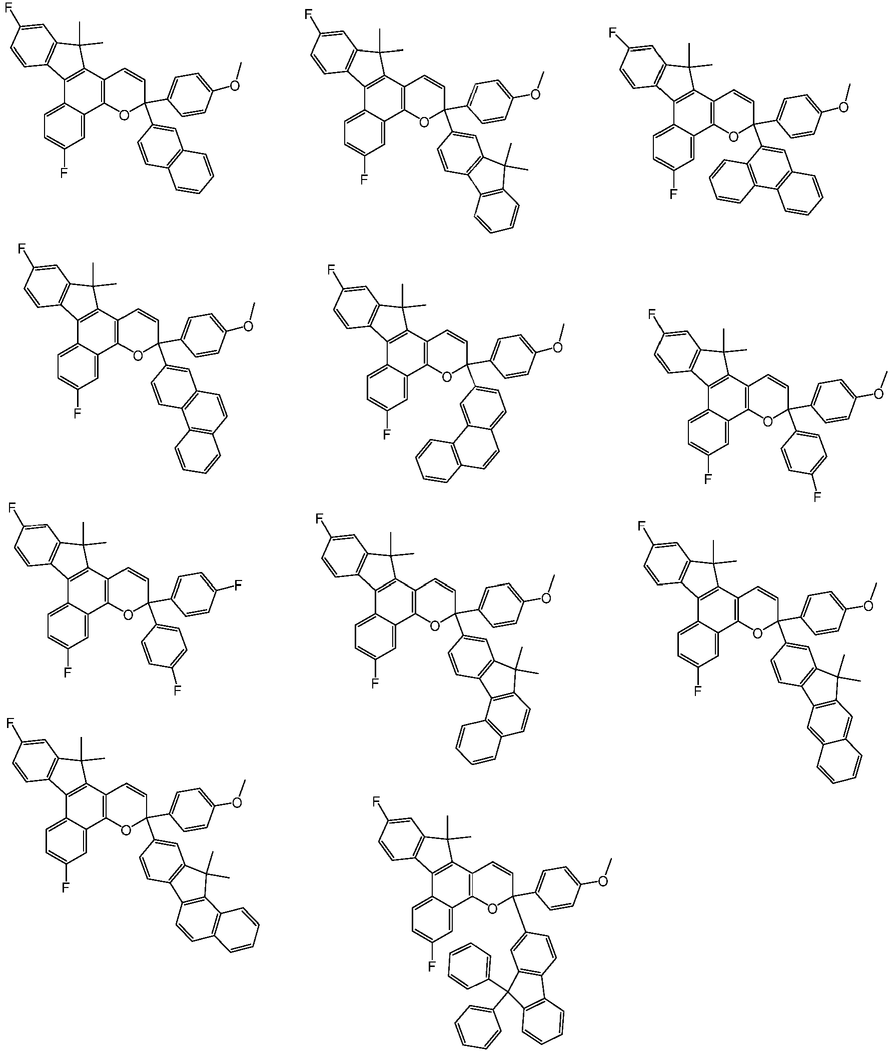

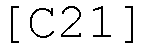

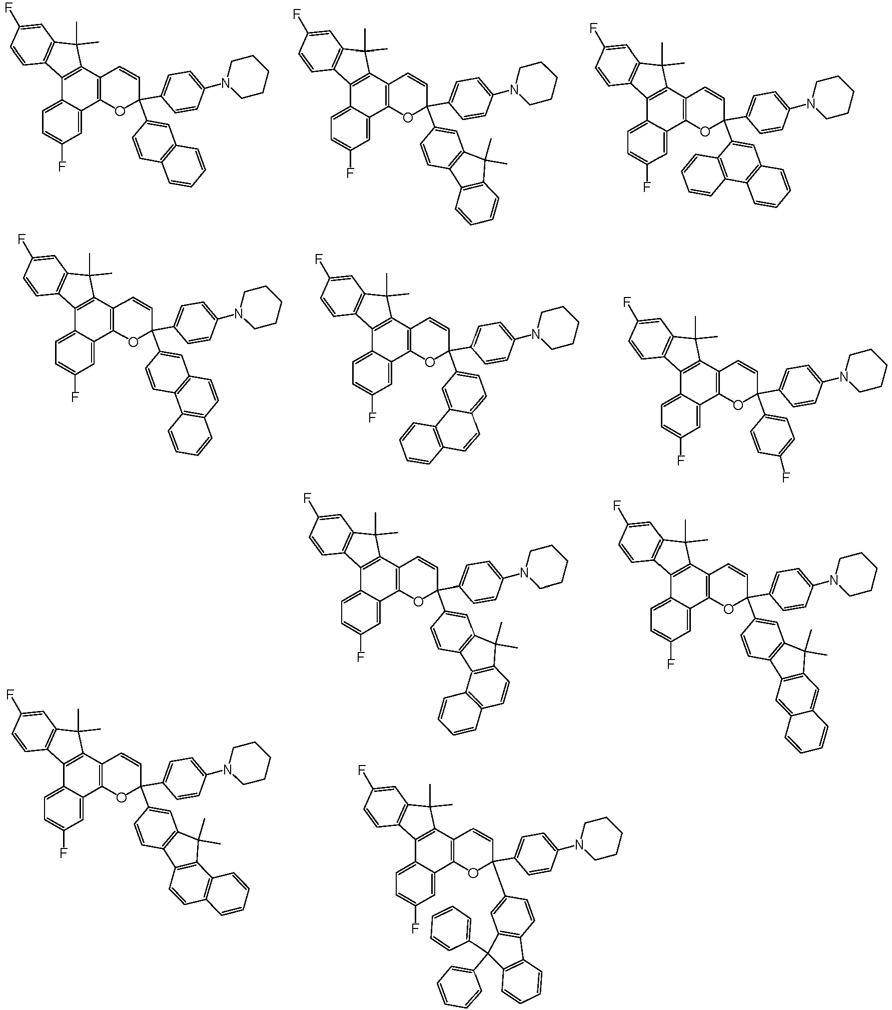

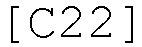

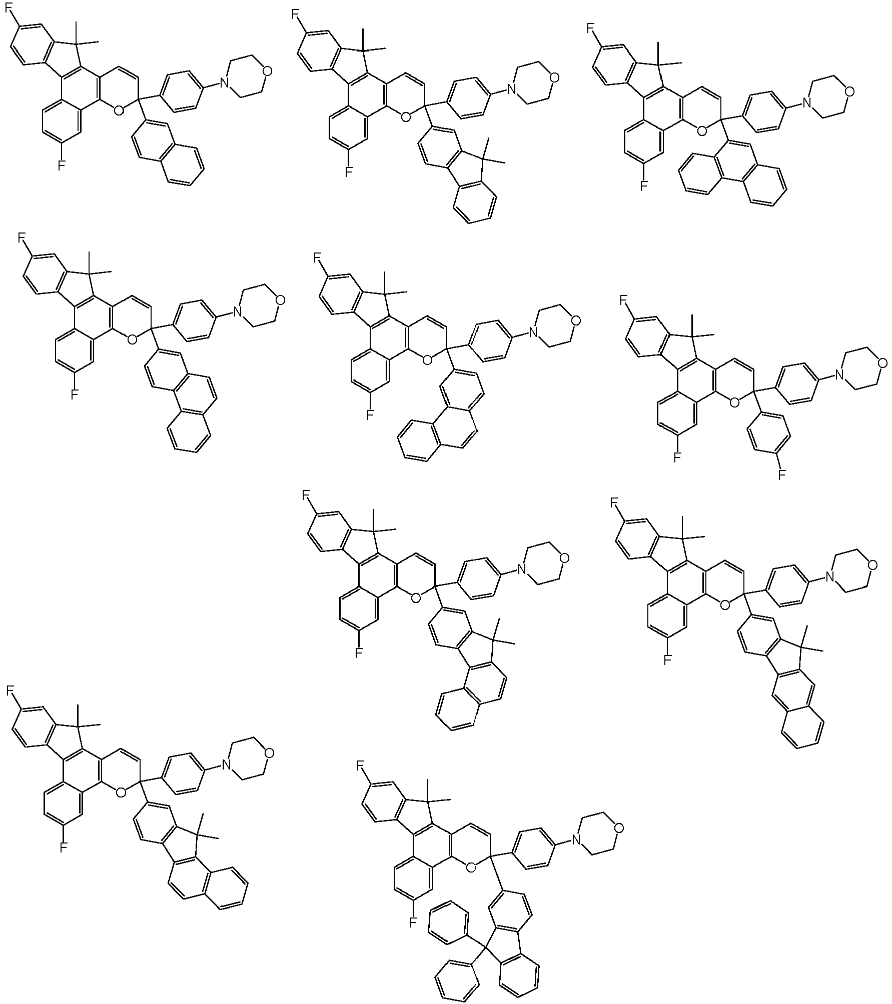

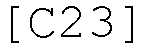

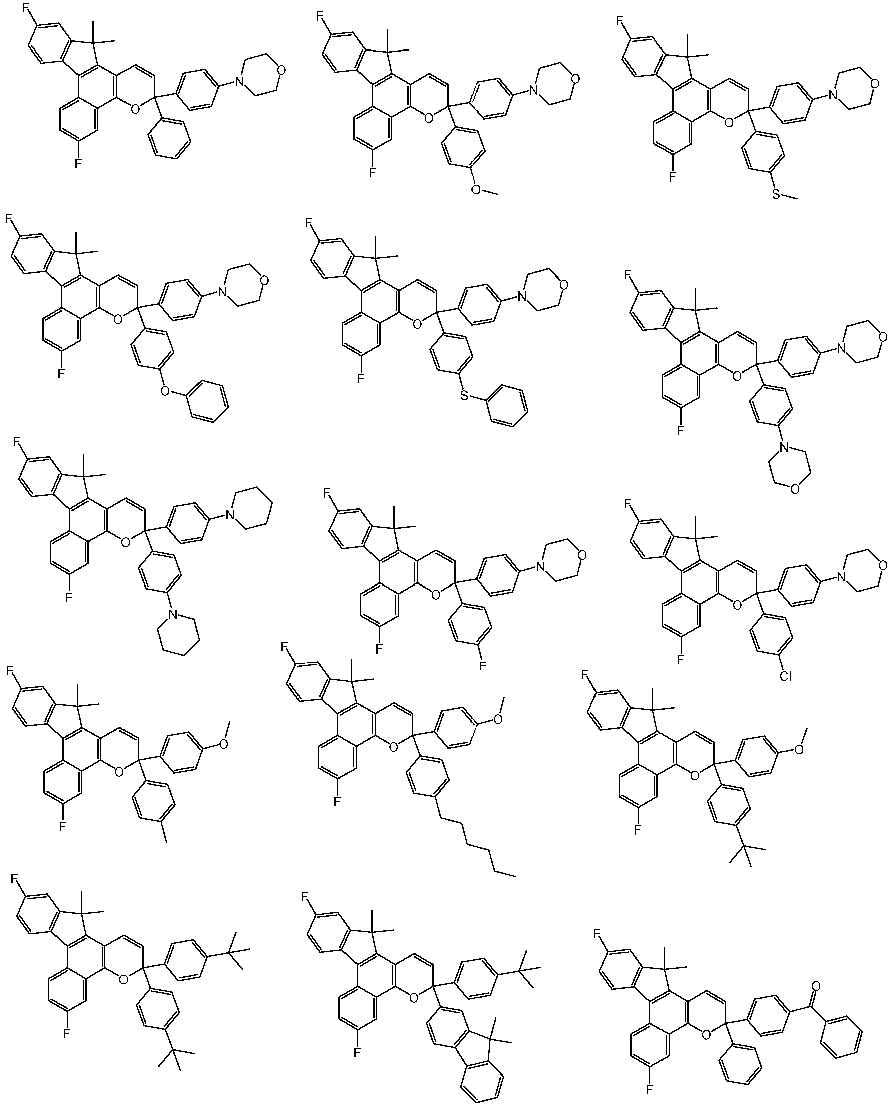

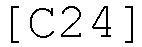

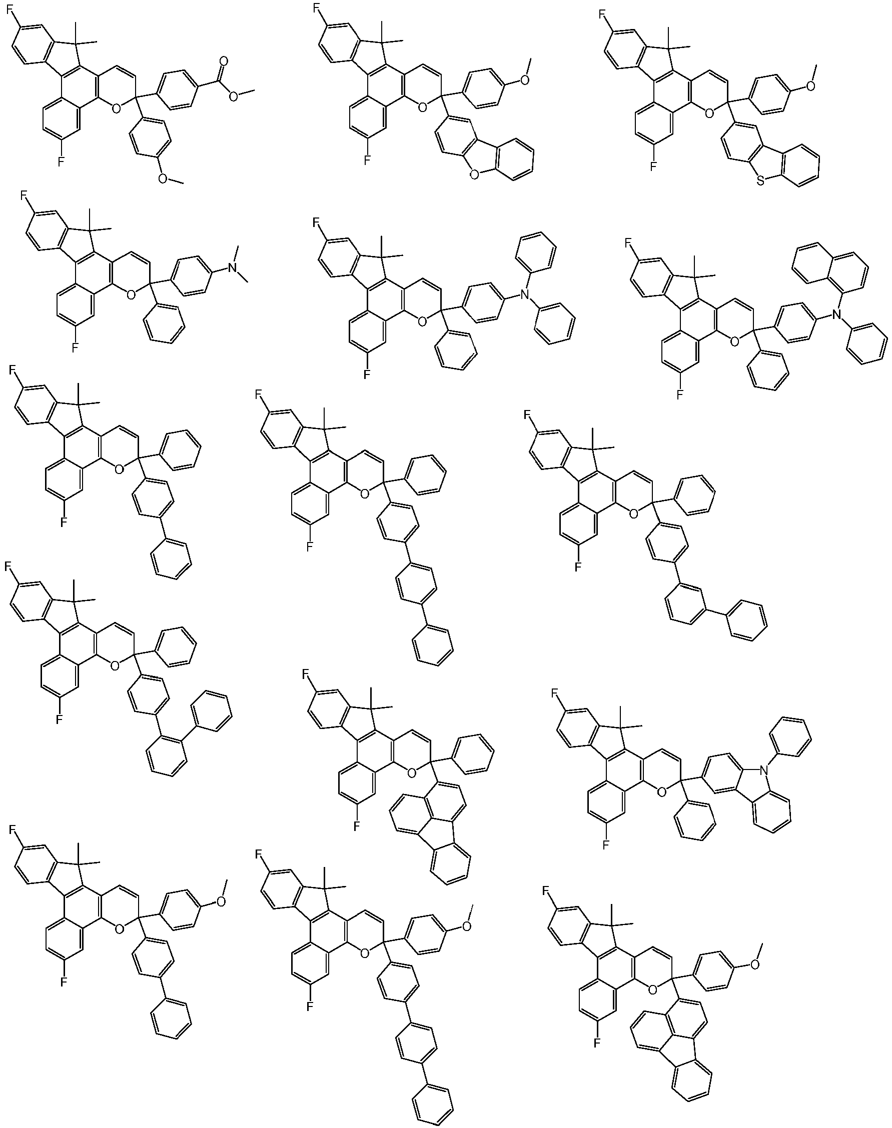

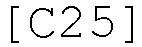

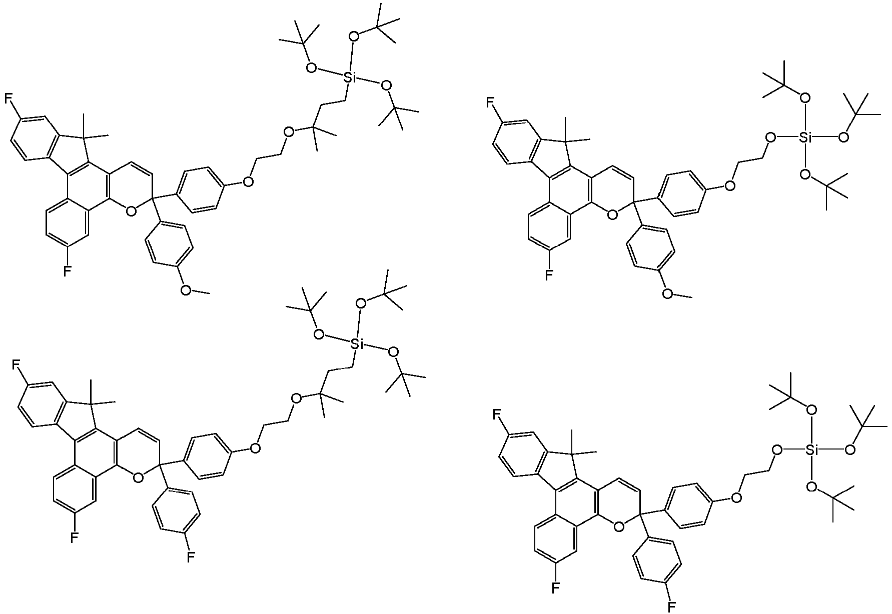

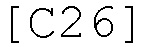

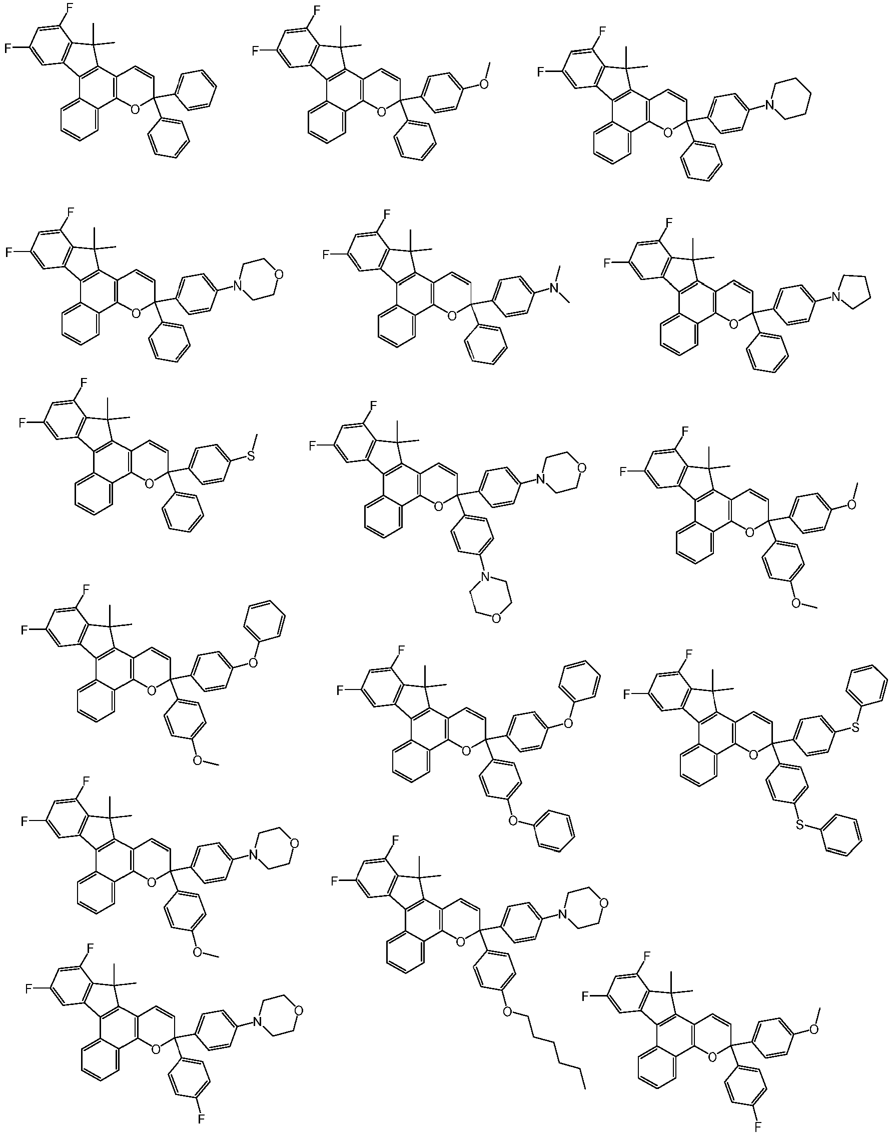

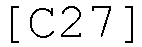

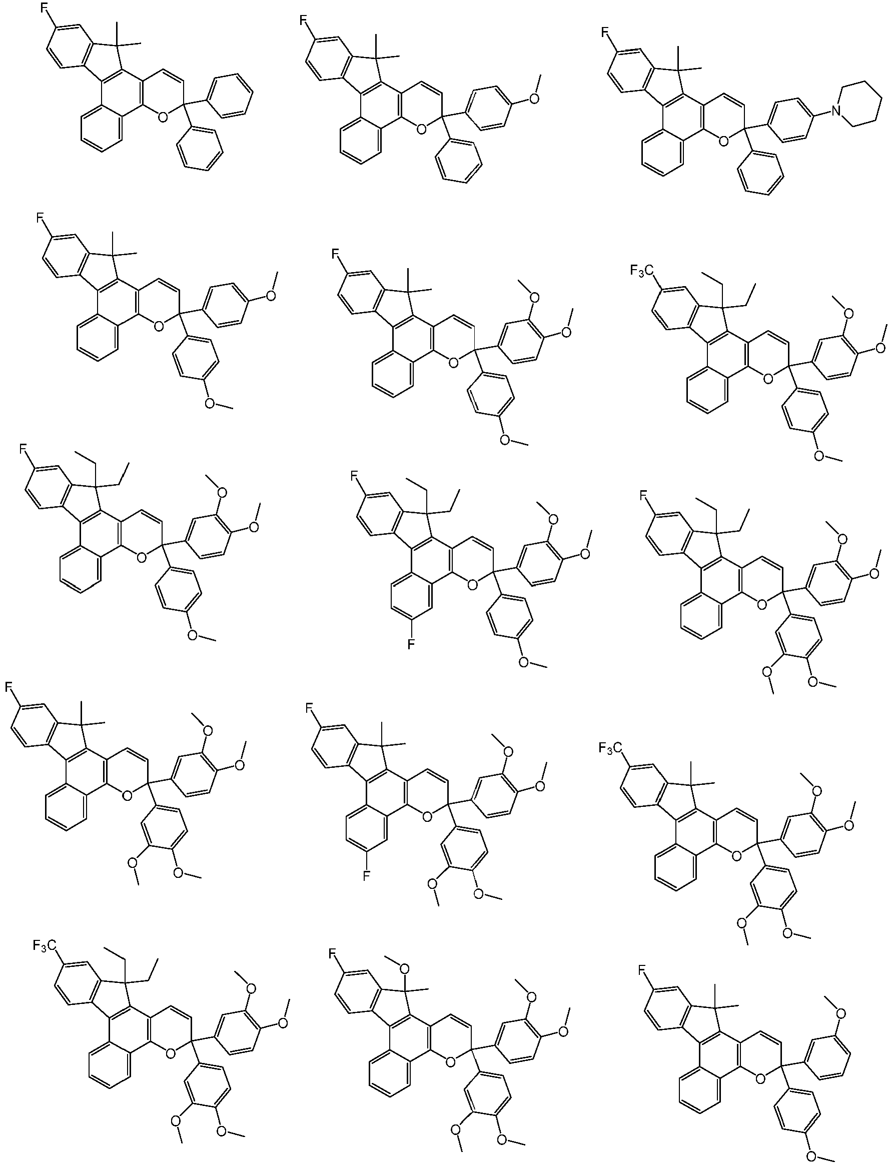

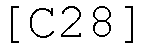

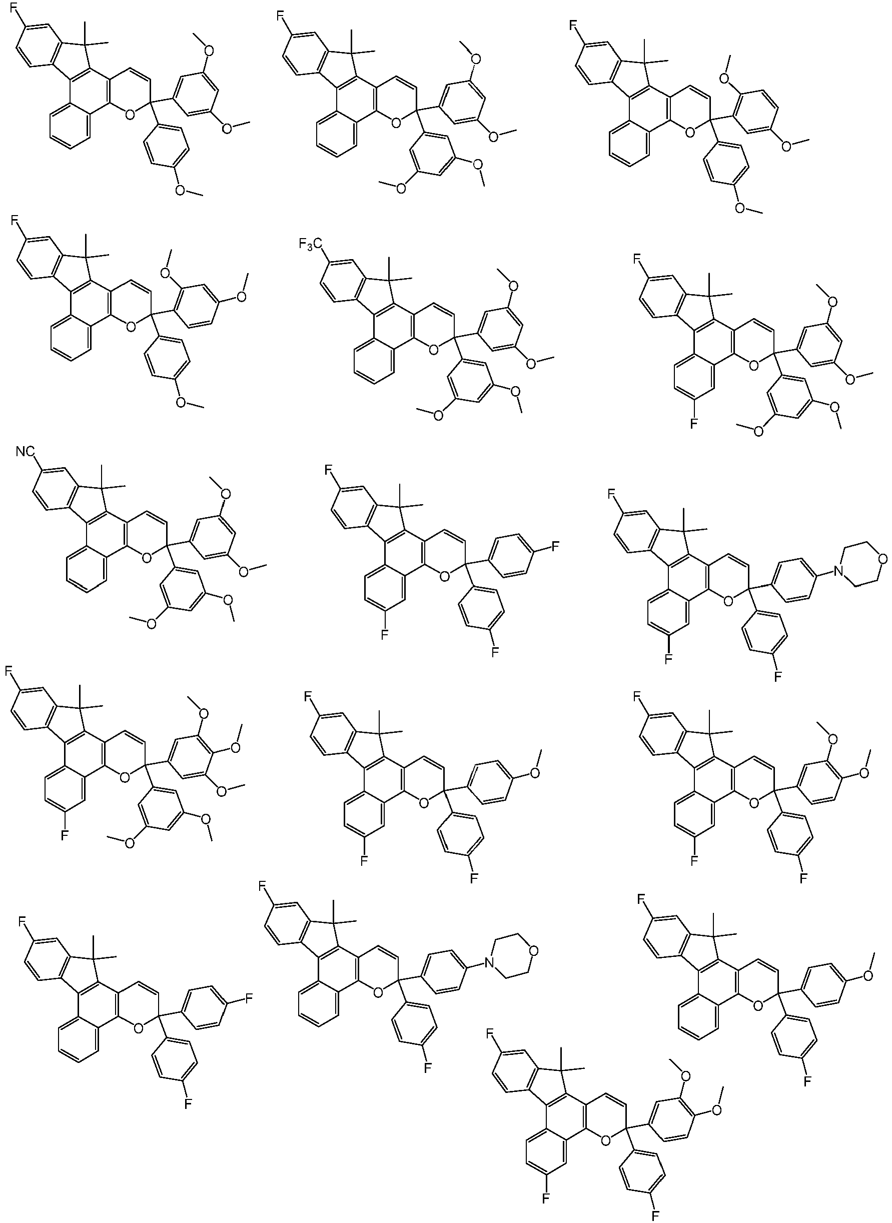

### <Compound Represented by General Formula B>

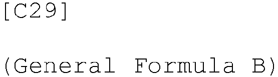

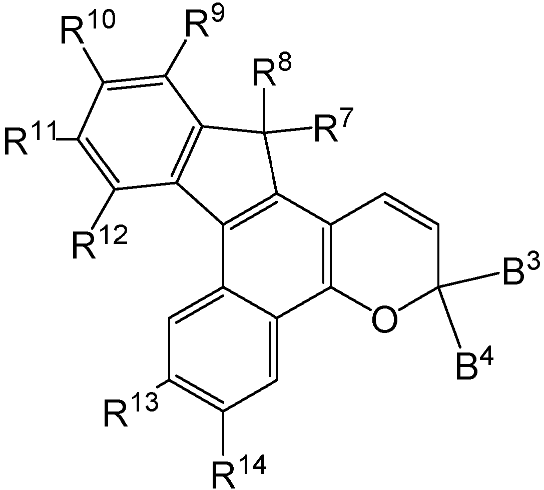

In General Formula B, R⁷ to R¹², B³, and B⁴ each independently represent a hydrogen atom or a substituent.

Preferably, R⁷ and R⁸ each independently represent a substituted or unsubstituted alkyl group having 1 to 20 carbon atoms, and more preferably, R⁷ and R⁸ each independently represent a methyl group, an ethyl group, a propyl group, a butyl group, a pentyl group, or a hexyl group. Further preferably, R⁷ and R⁸ each independently represent a methyl group or an ethyl group, and still further preferably, both R⁷ and R⁸ represent a methyl group or both R¹ and R² represent an ethyl group.

Preferably, B³ and B⁴ each independently represent a substituted or unsubstituted phenyl group. When the phenyl group has multiple substituents, two or more of these substituents may be bonded to form a ring. Specific examples of rings to be formed include rings included in exemplary compounds to be described later. The substitution position of a substituent in a substituted phenyl group is preferably a position that is the para-position with respect to the carbon atom to which B³ and B⁴ are bonded. Specific examples of substituents of the substituted phenyl group include substituents, which are included in exemplary compounds to be described later, such as a morpholino group, a piperidino group, a halogen atom, an alkoxy group, and the following substituents.

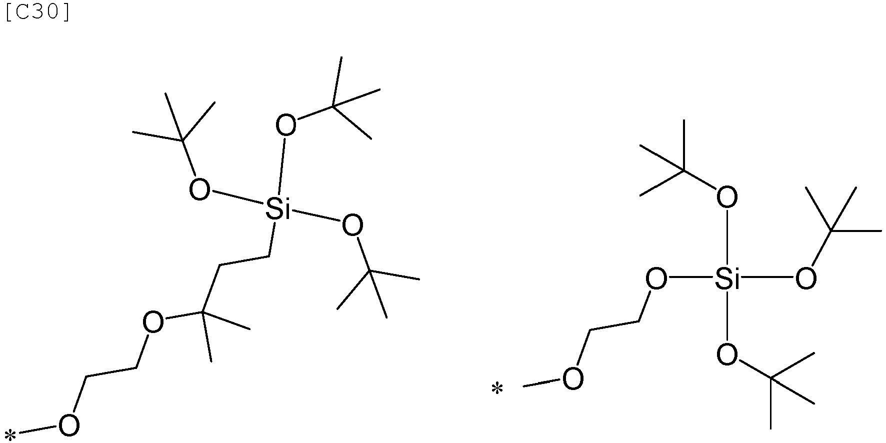

R⁹ to R¹² each independently represent a hydrogen atom or a substituent. In one aspect, R⁹ to R¹² can all be hydrogen atoms. In another aspect, R¹⁰ can be an electron-withdrawing group, and R⁹, R¹¹, and R¹² can all be hydrogen atoms. In another aspect, R⁹ and R¹¹ can each independently be an electron-withdrawing group, and R¹⁰ and R¹² can be hydrogen atoms. As the electron-withdrawing group, a halogen atom, a perfluoroalkyl group having 1 to 6 carbon atoms, a perfluorophenyl group, a perfluoroalkylphenyl group, or a cyano group is preferable. As the halogen atom, a fluorine atom is preferable. As the perfluoroalkyl group having 1 to 6 carbon atoms, a trifluoromethyl group is preferable.

In one aspect, R¹⁰ can be a substituted or unsubstituted phenyl group, and preferably, R²⁰ is a substituted or unsubstituted phenyl group and R⁹, R¹¹, and R¹² can be hydrogen atoms. Specific examples of such substituted phenyl groups include a phenyl group that has been substituted with one or more halogen atoms and/or one or more cyano groups, for example, a phenyl group in which all five substitution positions of the phenyl group have been substituted with halogen atoms (preferably fluorine atoms), and a monosubstituted phenyl group in which a position that is the para-position with respect to the carbon atom to which R¹⁰ is bonded has been substituted with a cyano group.

R¹³ and R¹⁴ each independently represent a hydrogen atom or an electron-donating group. However, one or more of R¹³ and R¹⁴ represent an electron-donating group. It is preferable that R¹³ and R¹⁴ each independently represent an electron-donating group selected from the group consisting of a methoxy group, an ethoxy group, a phenoxy group, a methylsulfide group, a phenylsulfide group, a dimethylamino group, a pyrrolidino group, a piperidino group, a morpholino group, and a thiomorpholino group.

In one aspect, one of R¹³ and R¹⁴ can be a hydrogen atom and the other can be an electron-donating group, and in another aspect, both can each independently be an electron-donating group. Both R¹³ and R¹⁴ are preferably electron-donating groups. In this case, R¹³ and R¹⁴ can be the same or different electron-donating groups. Among them, for R¹³ and R¹⁴, it is preferable that R¹³ be a morpholino group and R¹⁴ be an alkoxy group (preferably a methoxy group), that R¹³ be a morpholino group and R¹⁴ be a methylsulfide group (-S-CH₃), that both R¹³ and R¹⁴ be alkoxy groups (preferably methoxy groups), and that both R¹³ and R¹⁴ be methylsulfide groups (-S-CH₃).

Examples of the compounds represented by General Formula B include the following compounds. However, the present invention is not limited to the compounds exemplified below.

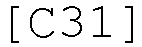

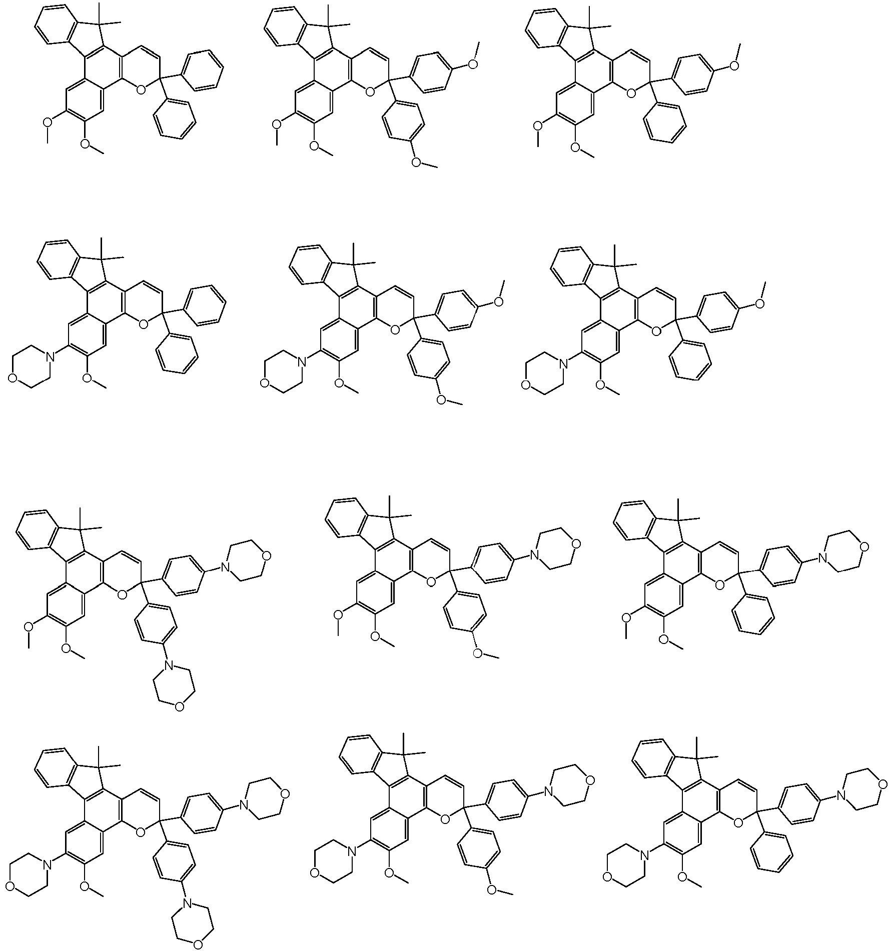

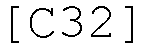

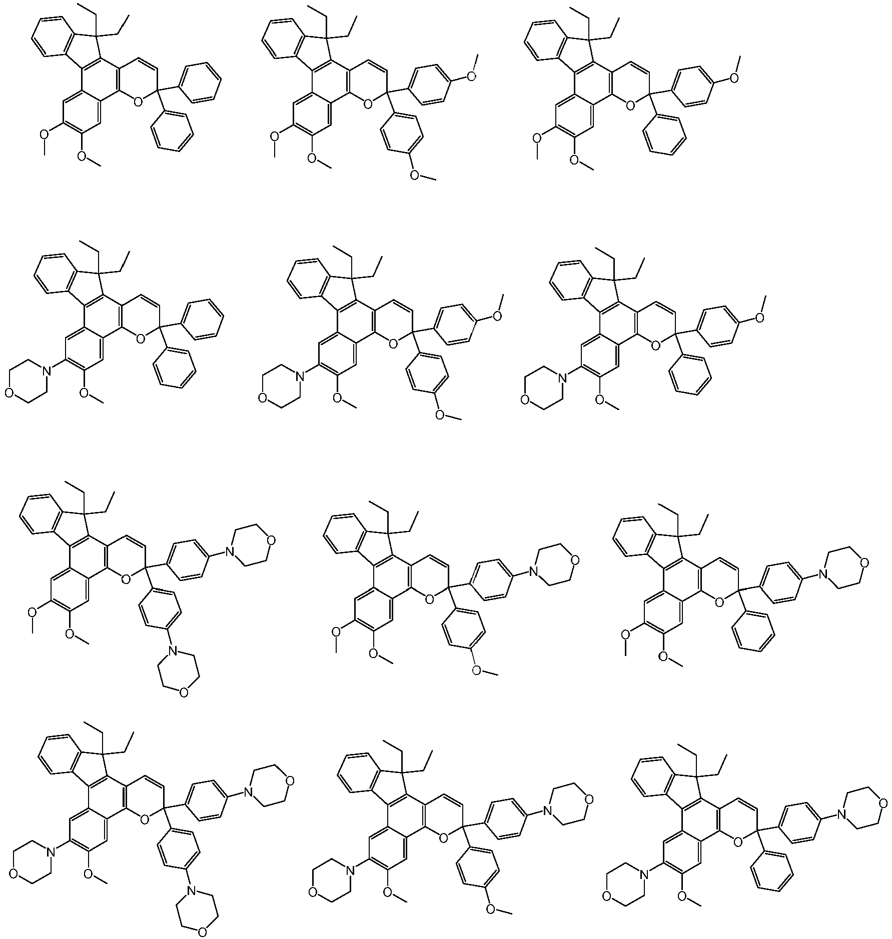

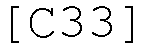

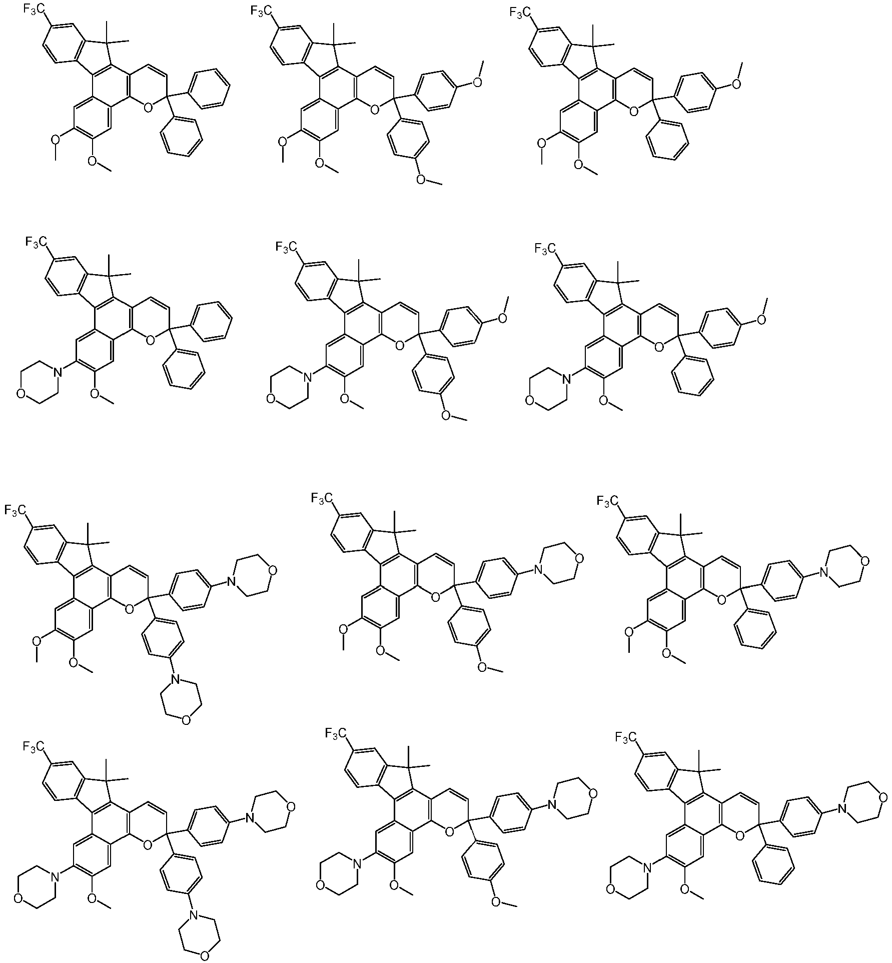

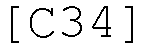

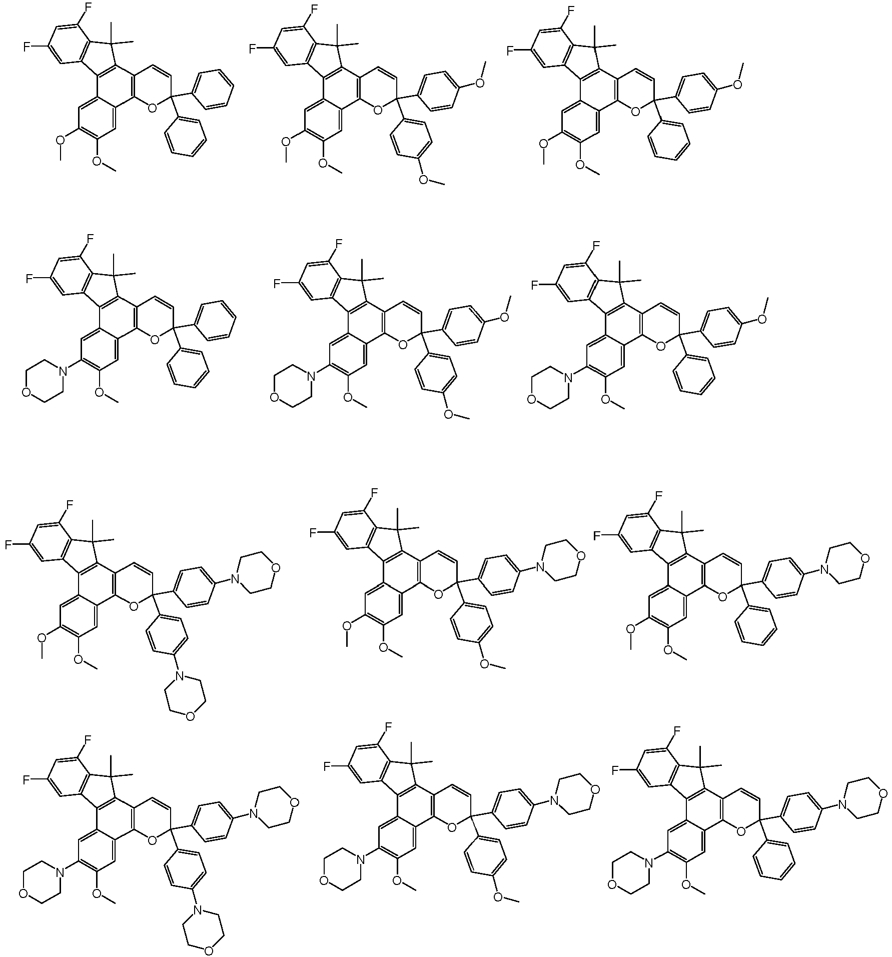

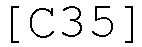

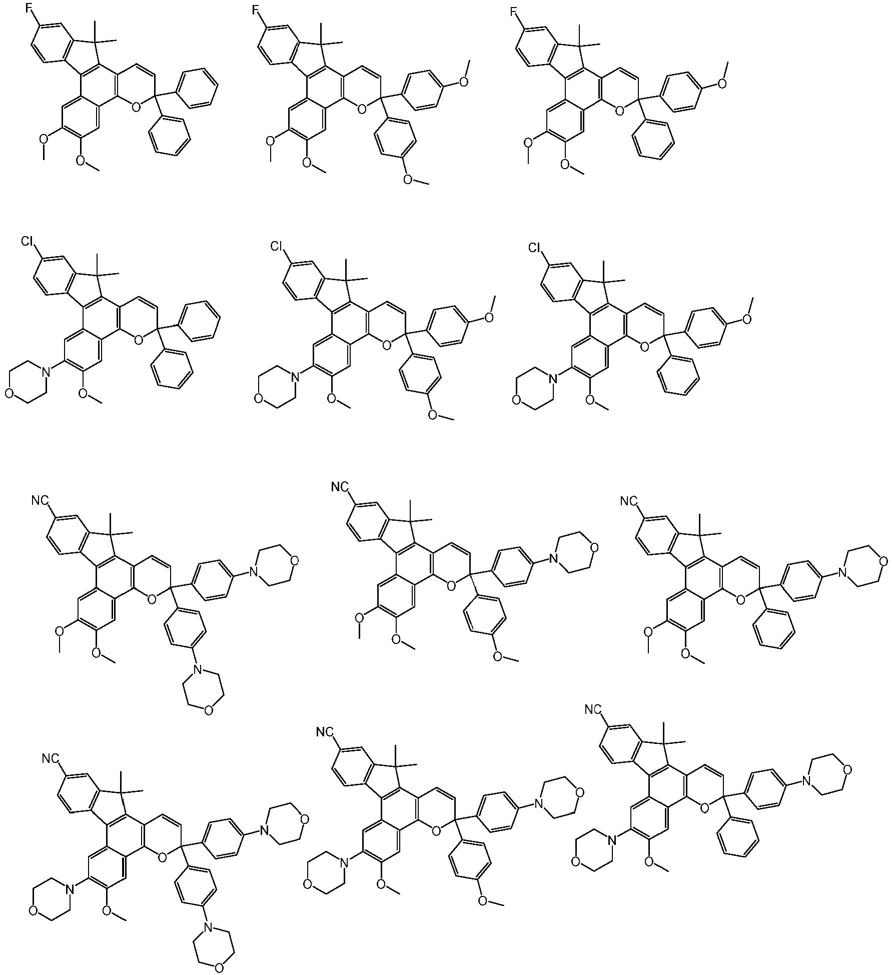

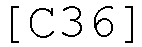

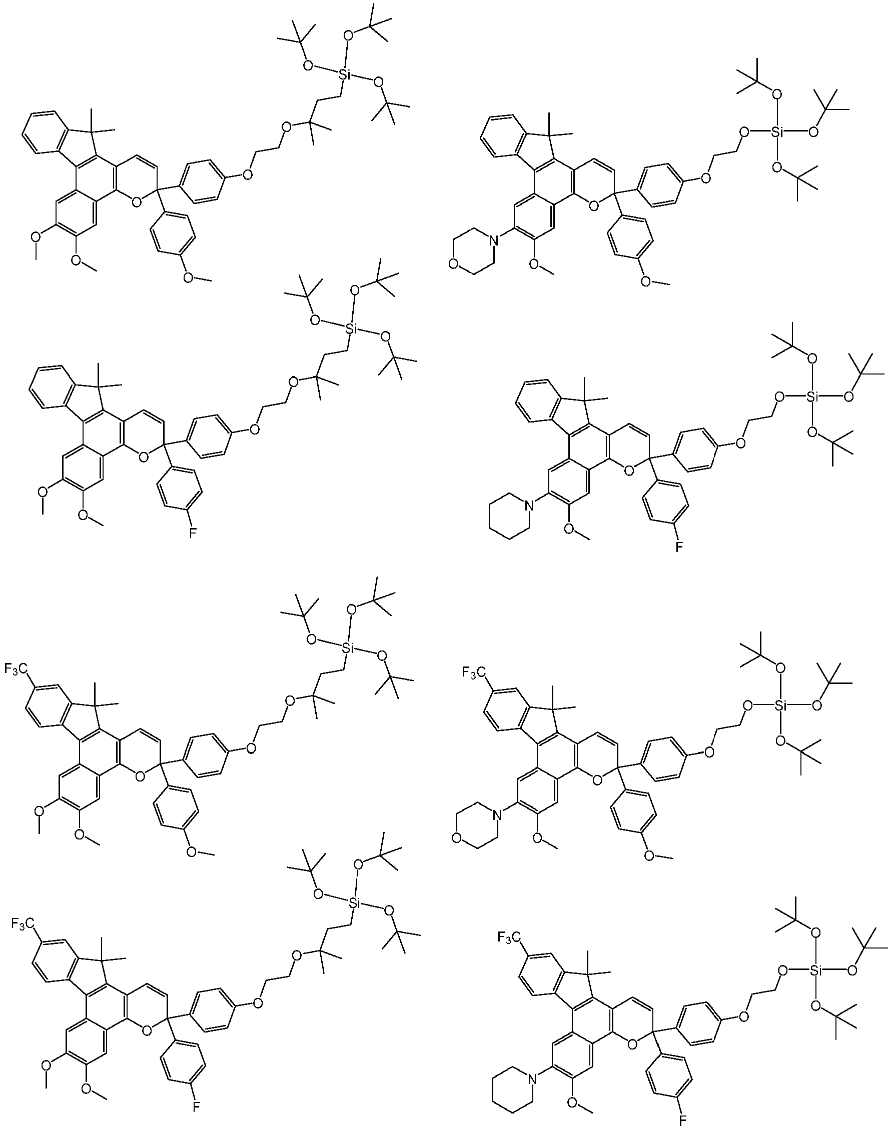

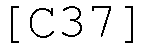

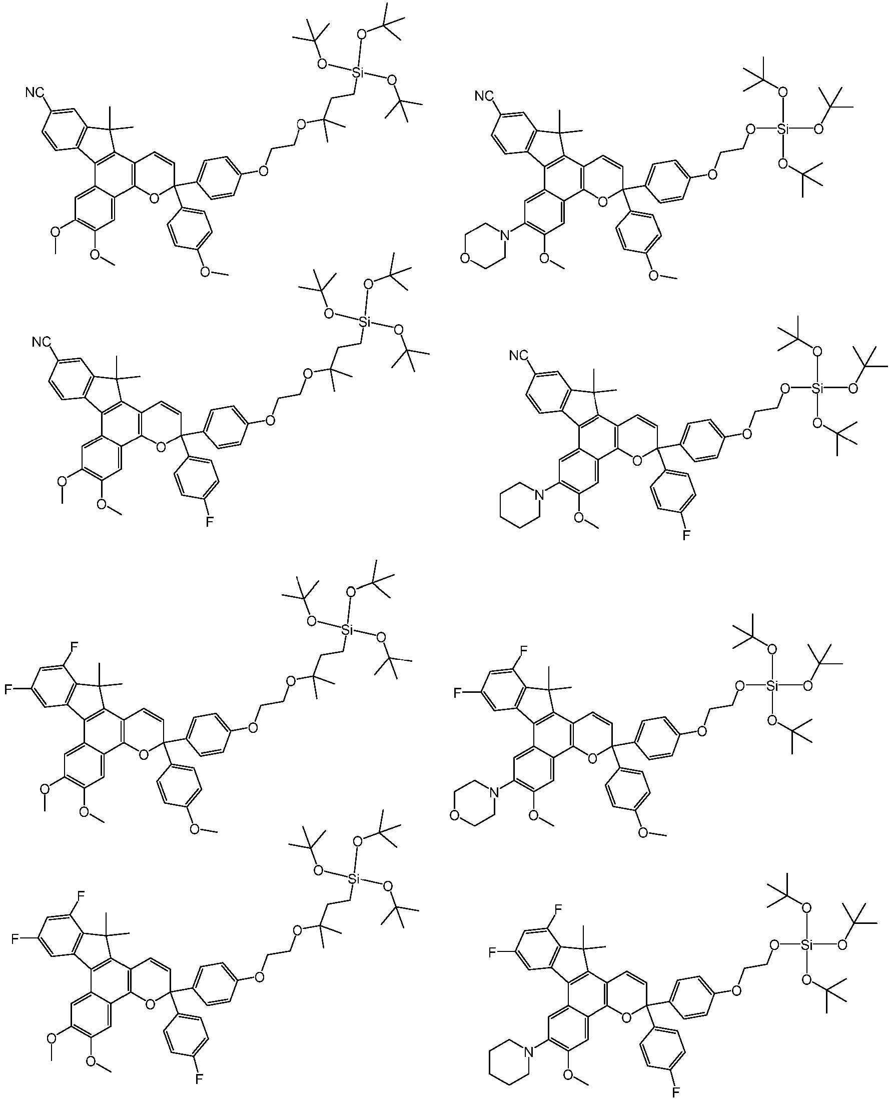

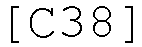

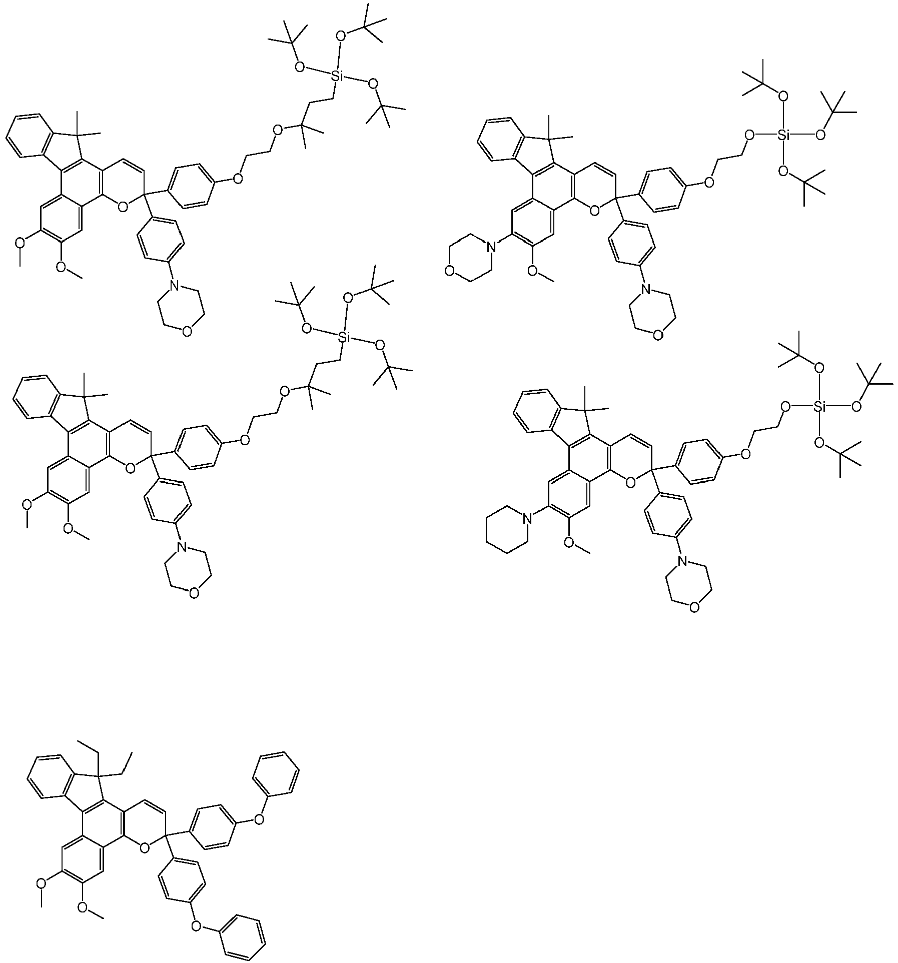

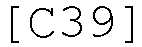

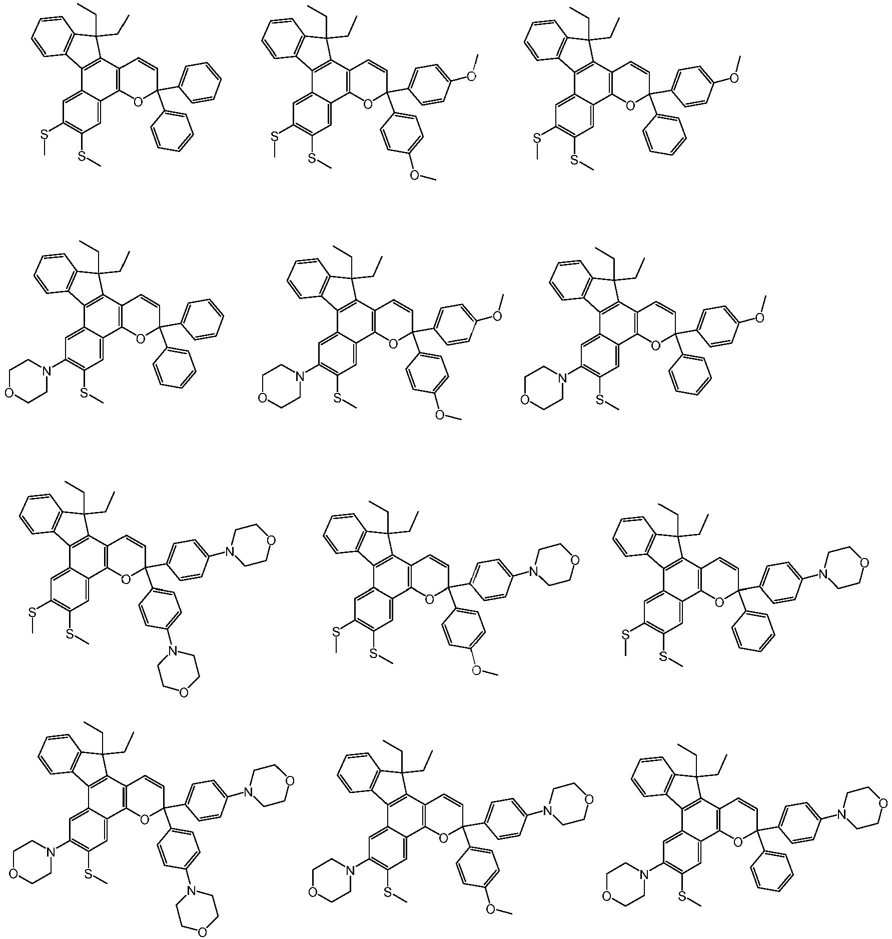

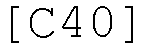

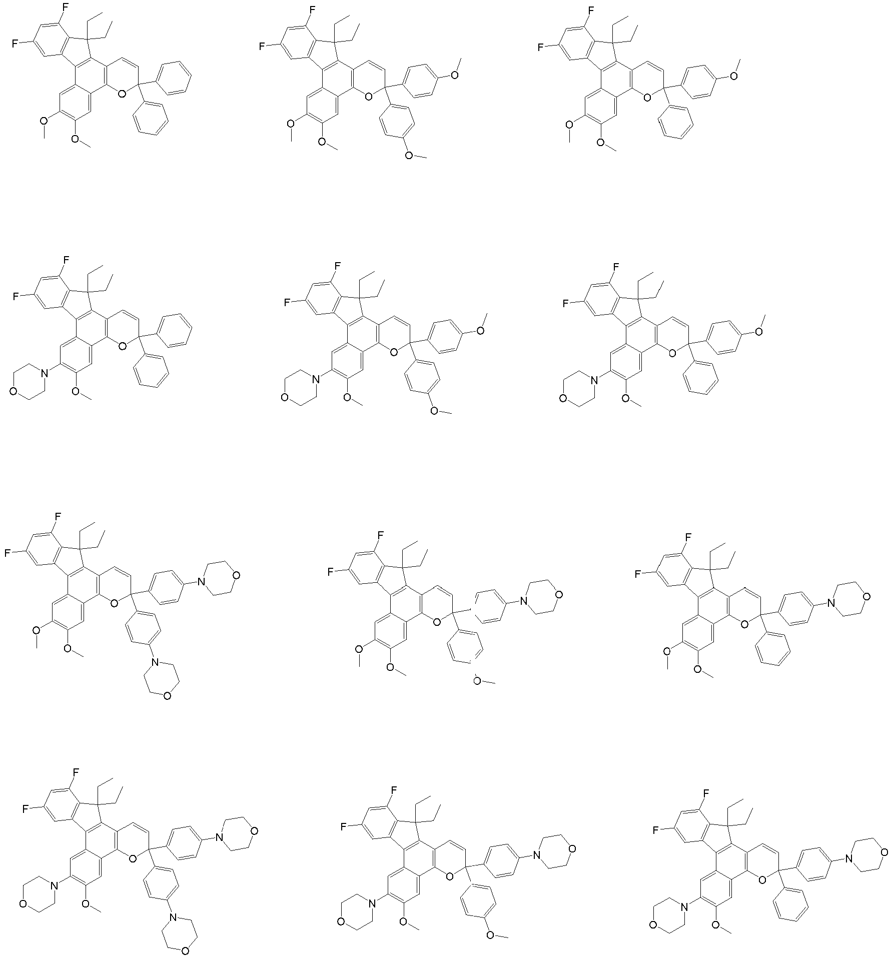

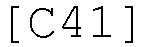

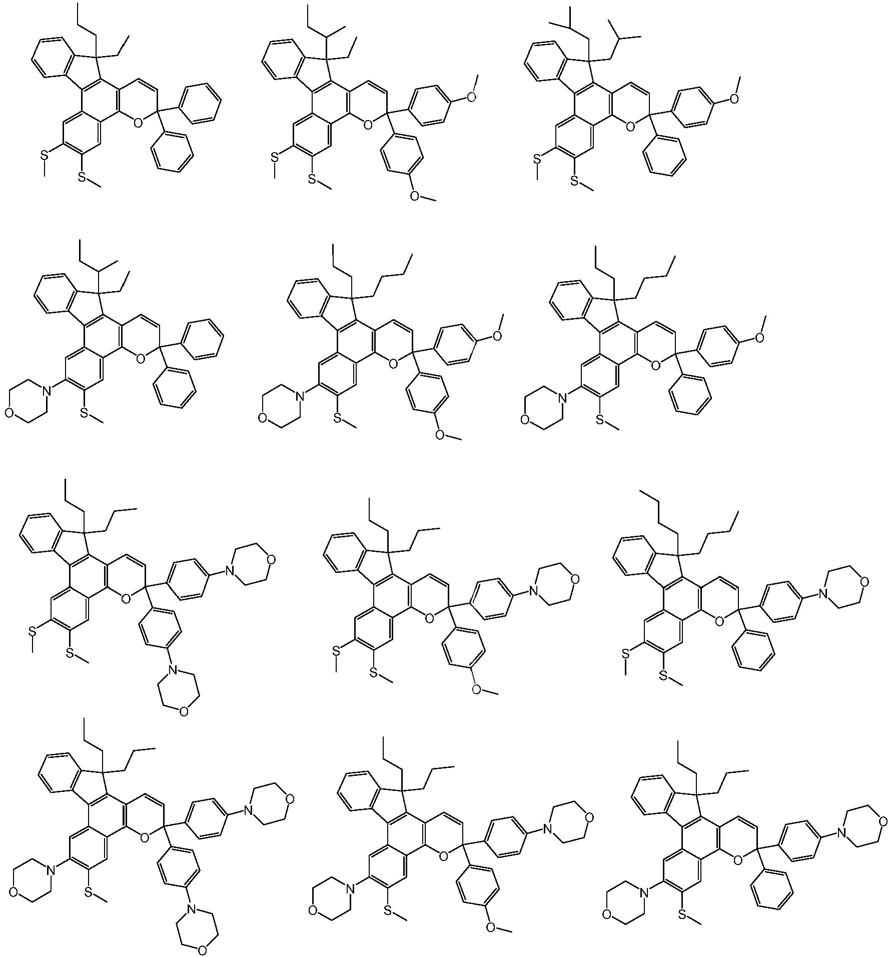

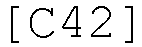

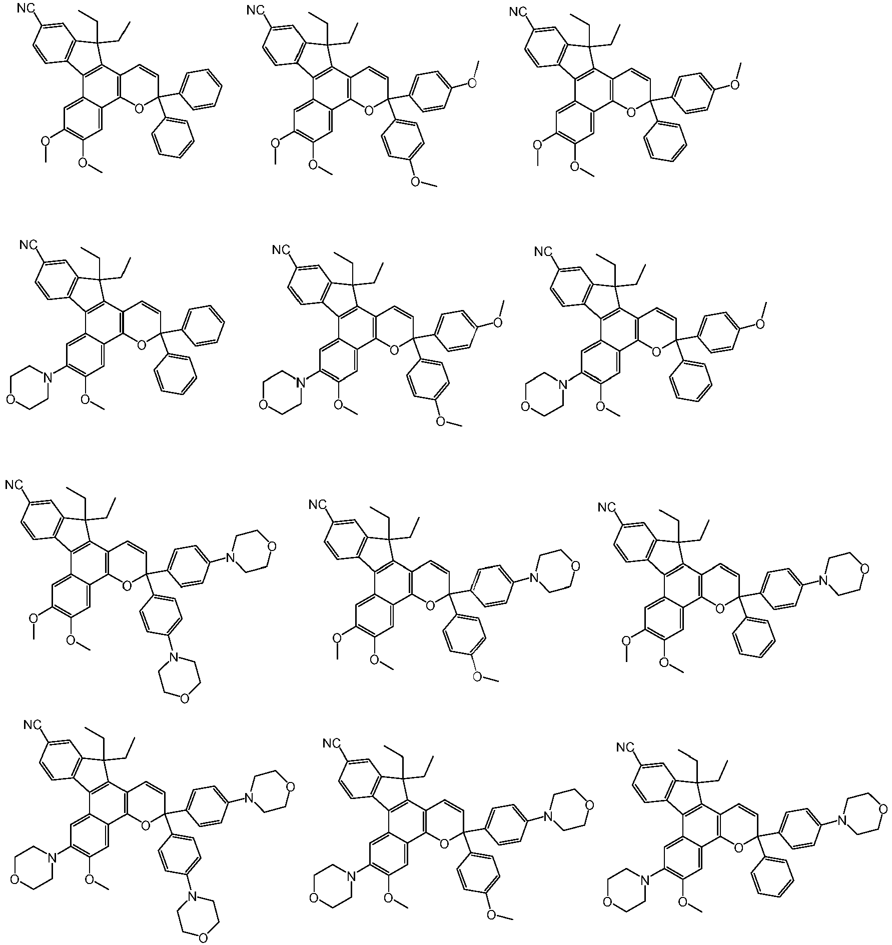

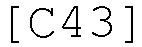

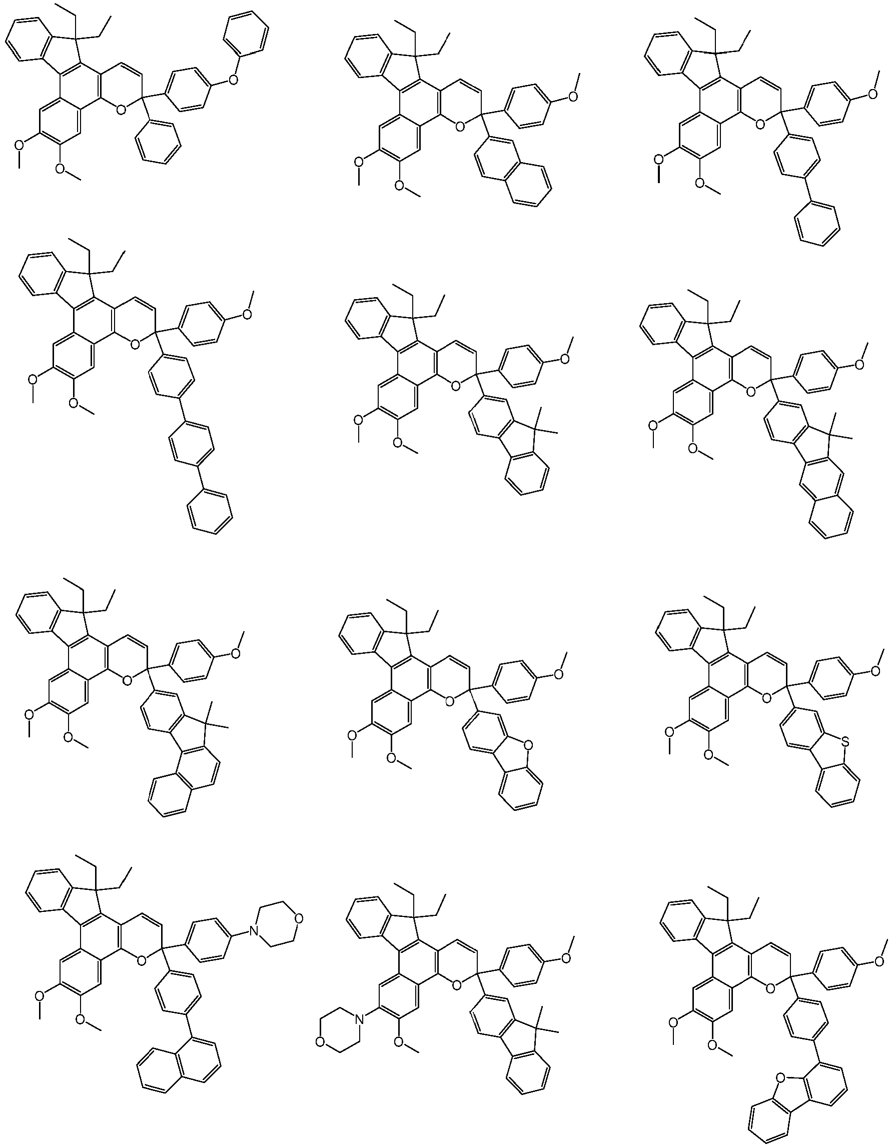

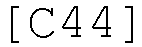

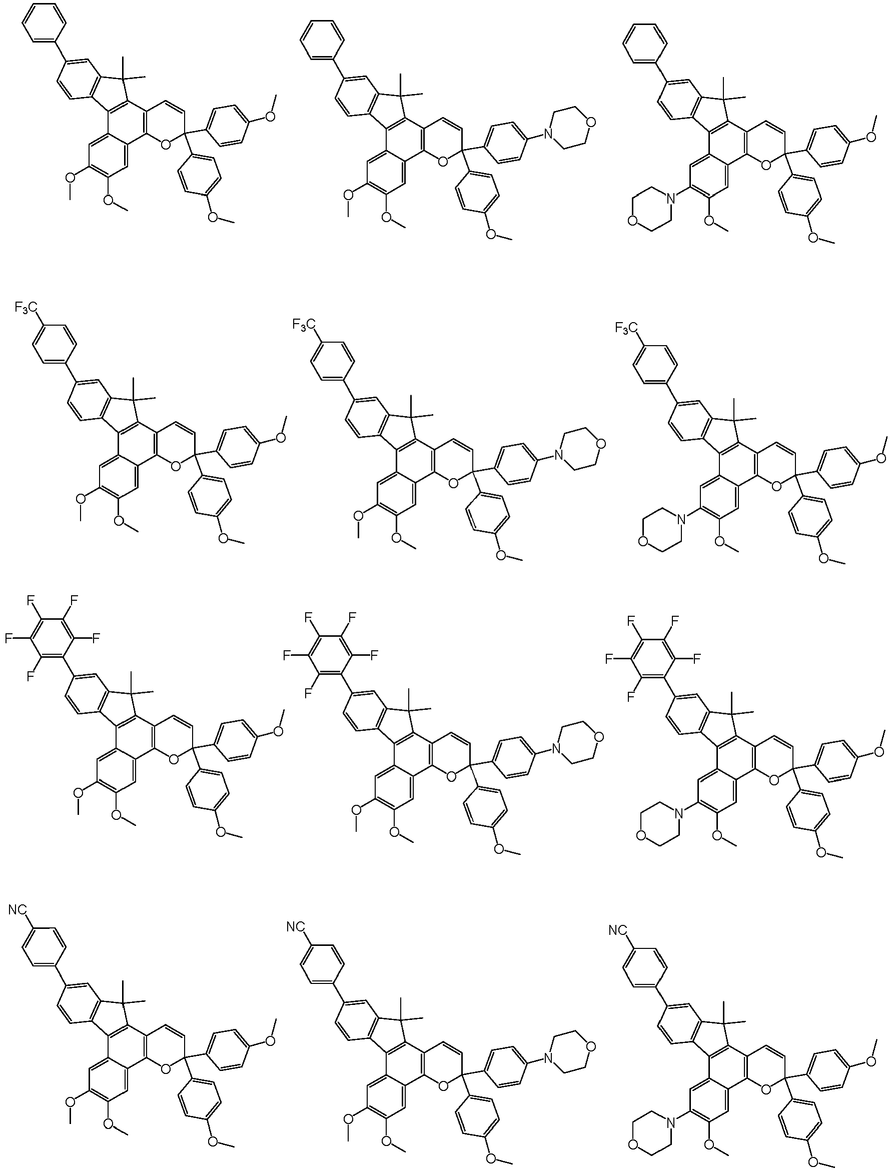

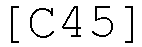

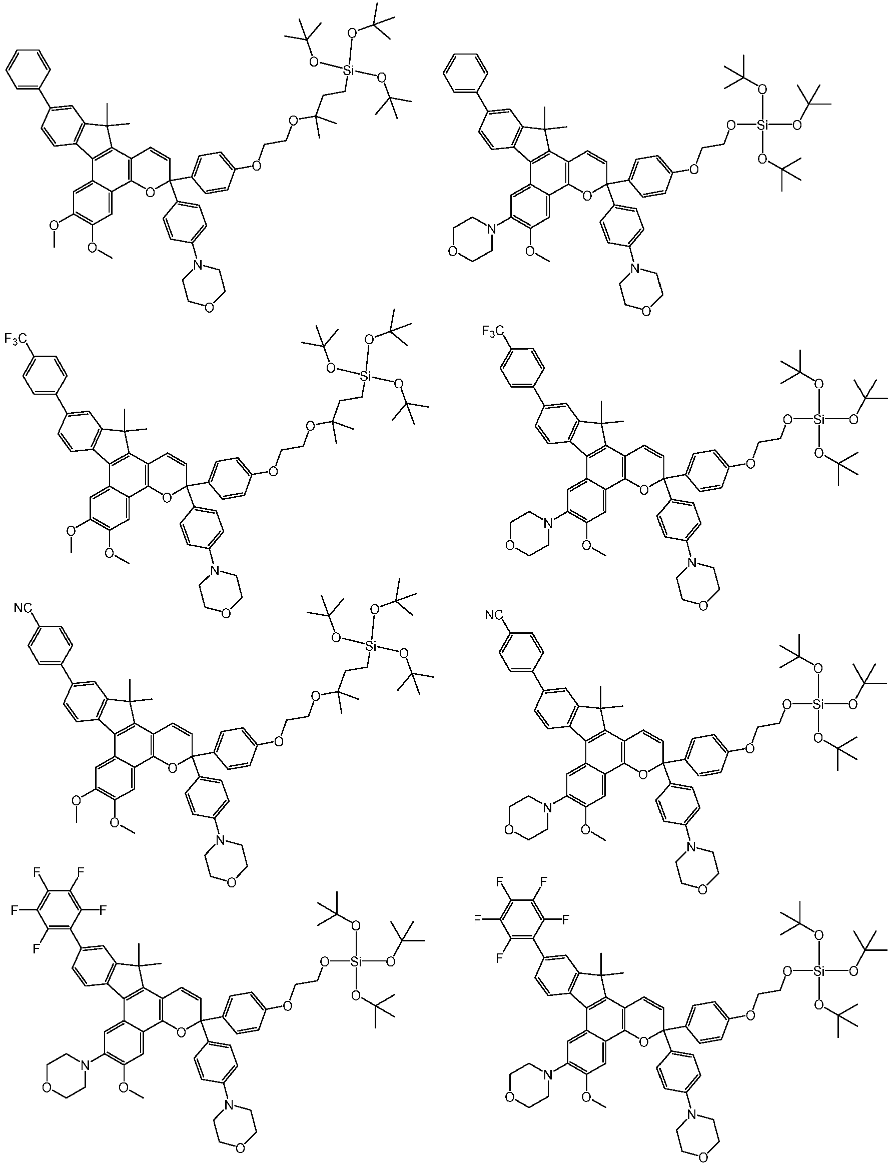

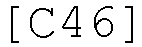

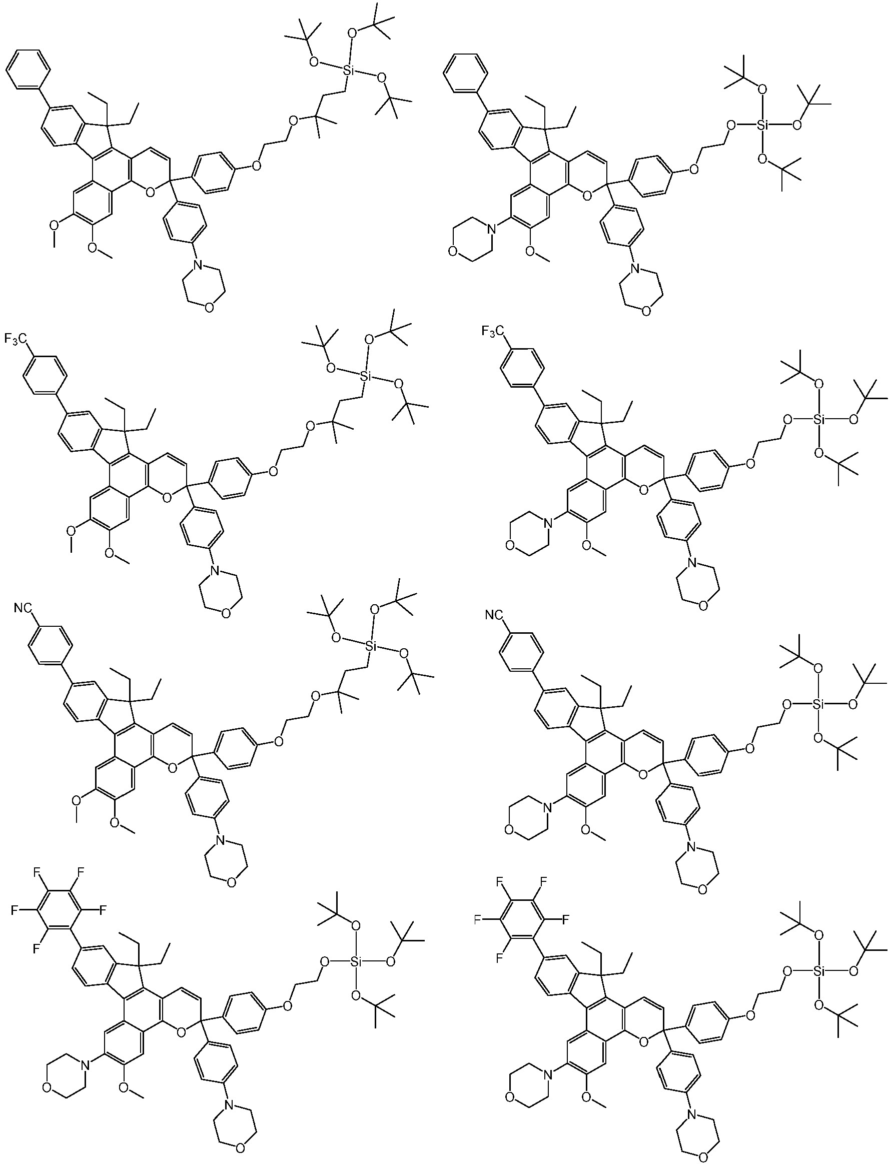

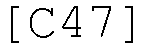

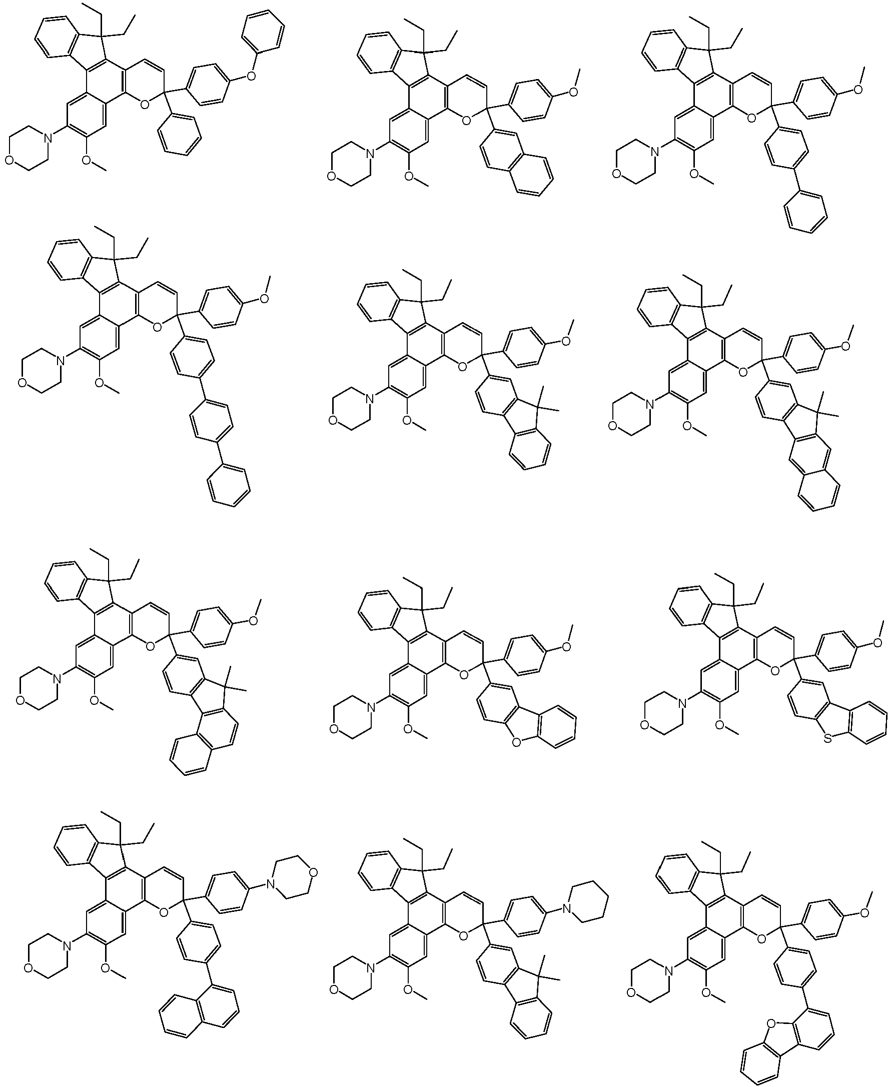

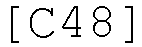

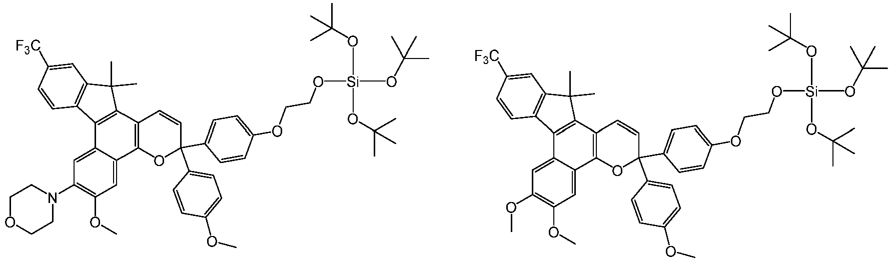

The compound represented by General Formula A and the compound represented by General Formula B can be synthesized by a known method. For a synthesis method, the following documents can be referred to, for example. Japanese Patent No. 4884578, US 2006/0226402 A1, US 2006/0228557 A1, US 2008/0103301 A1, US 2011/0108781 A1, US 2011/0108781 A1, U.S. Patent No. 7527754, U.S. Patent No. 7556751, WO 2001/60811 A1, WO 2013/086248 A1, WO 1996/014596 A1, and WO 2001/019813 A1.

The photochromic article according to one aspect of the present invention and the photochromic composition according to one aspect of the present invention contain one or more compounds represented by General Formula A and one or more compounds represented by General Formula B.

For the compounds represented by General Formula A contained in the above-mentioned photochromic article and the above-mentioned photochromic composition, one type can be used alone, or two or more types (for example, two or more and four or less) can be used.

The compounds represented by General Formula B contained in the above-mentioned photochromic article and the above-mentioned photochromic composition, one type can be used alone, two or more types can be used, but two or more types are preferable, and for example, four or less or three or less types can be used.

In the above-mentioned photochromic article and the above-mentioned photochromic composition, the compound represented by General Formula B is preferably more than the compound represented by General Formula A on a mass basis. When the total of the compound represented by General Formula A and the compound represented by General Formula B is 100 mass%, the content of the compound represented by General Formula B is preferably more than 50 mass%, more preferably 60 mass% or more, further preferably 70 mass% or more, still further preferably 80 mass% or more, and even further preferably 90 mass% or more. With respect to the total (100 mass%) of the compound represented by General Formula A and the compound represented by General Formula B, the content of the compound represented by General Formula B can be less than 100 mass%, and can be 99 mass% or less, 98 mass% or less, 97 mass% or less, 96 mass% or less, or 95 mass% or less. When the above-mentioned photochromic article and the above-mentioned photochromic composition contain two or more types of the compounds represented by General Formula B, the content of the compound represented by General Formula B is the total content of these compounds. The same also applies to the contents of various components in the present invention and the present specification.

In the above-mentioned photochromic article and the above-mentioned photochromic composition, when the total amount of the photochromic article and the photochromic composition is 100 mass%, the total content of the compound represented by General Formula A and the compound represented by General Formula B can be about 0.1 to 15.0 mass%, for example. However, the above-mentioned total content is not limited to this range.

### [Photochromic Article and Photochromic Composition]

The above-mentioned photochromic article can have at least a substrate. In one aspect, the compound represented by General Formula A and the compound represented by General Formula B can be contained in the substrate of the above-mentioned photochromic article. The above-mentioned photochromic article can have the substrate and a photochromic layer, and the compound represented by General Formula A and the compound represented by General Formula B can be contained in the substrate and/or the photochromic layer. Regarding the substrate and the photochromic layer, the compound represented by General Formula A and the compound represented by General Formula B can be contained only in the substrate in one aspect, can be contained only in the photochromic layer in another aspect, or can be contained in the substrate and the photochromic layer in still another aspect. In addition, in the substrate and the photochromic layer, only the compound represented by General Formula A and the compound represented by General Formula B can be contained as photochromic compounds, or one or more of other photochromic compounds can also be contained. Examples of the other photochromic compounds include azobenzenes, spiropyrans, spirooxazines, naphthopyrans, indenonaphthopyrans, phenanthropyrans, hexaarylbisimidazoles, donor-acceptor Stenhouse adducts (DASA), salicylidene anilines, dihydropyrenes, anthracene dimers, fulgides, diarylethenes, phenoxynaphthacenequinones, and stilbenes.

### <Substrate>

The above-mentioned photochromic article can contain a substrate selected according to the type of photochromic article. Examples of substrates include plastic lens substrates and glass lens substrates as spectacle lens substrates. The glass lens substrate can be a lens substrate made of inorganic glass, for example. Examples of plastic lens substrates include a styrene resin including a (meth)acrylic resin; an allyl carbonate resin such as a polycarbonate resin, an allyl resin, and a diethylene glycol bisallyl carbonate resin (CR-39); a vinyl resin; a polyester resin; polyether resin; a urethane resin obtained by reacting an isocyanate compound with a hydroxy compound such as diethylene glycol; a thiourethane resin obtained by reacting an isocyanate compound with a polythiol compound; and a cured product (generally called a transparent resin) obtained by curing a curable composition containing a (thio)epoxy compound having one or more disulfide bonds in the molecule. As the lens substrate, an undyed one (colorless lens) may be used, or a dyed one (dyed lens) may be used. The refractive index of the lens substrate can be about 1.50 to 1.75, for example. However, the refractive index of the lens substrate is not limited to the above-mentioned range, and the refractive index may be within the above-mentioned range or may be deviated from the above-mentioned range by plus or minus increment. The refractive index herein refers to the refractive index for light having a wavelength of 500 nm. In addition, the lens substrate may be a lens having refractive power (so-called prescription lens), or may be a lens not having refractive power (so-called non-prescription lens).

For example, the above-mentioned photochromic composition can be a polymerizable composition. In the present invention and the present specification, the term "polymerizable composition" is a composition containing one or more polymerizable compounds. By forming a polymerizable composition containing at least one or more compounds represented by General Formula A, one or more compounds represented by General Formula B, and one or more polymerizable compounds by a known forming method, a cured product of such a polymerizable composition can be produced. Such a cured product can be contained as a substrate in the above-mentioned photochromic article and/or can be contained as a photochromic layer therein. A curing treatment can be an irradiation with light and/or heat treatment. The polymerizable compound is a compound having a polymerizable group, and as the polymerization reaction of the polymerizable compound proceeds, the polymerizable composition is cured, and thereby a cured product can be formed. The polymerizable composition can further contain one or more additives (for example, a polymerization initiator or the like).

Spectacle lenses can be various lenses such as monofocal lenses, multifocal lenses, and progressive power lenses. The type of lenses is determined by the surface shape of both surfaces of a lens substrate. In addition, the surface of the lens substrate may be any of a convex surface, a concave surface, and a flat surface. In a normal lens substrate and a spectacle lens, the object-side surface is a convex surface, and the eyeball-side surface is a concave surface. However, there is no particular limitation. The photochromic layer can usually be provided on the object-side surface of the lens substrate, but may be provided on the eyeball-side surface.

### <Photochromic Layer>

The photochromic layer can be a layer that is provided directly on the surface of the substrate or provided indirectly via one or more other layers. The photochromic layer can be, for example, a cured layer obtained by curing a polymerizable composition. The photochromic layer can be formed as a cured layer obtained by curing a polymerizable composition containing at least one or more compounds represented by General Formula A, one or more compounds represented by General Formula B, and one or more polymerizable compounds. For example, by directly applying such a polymerizable composition onto the surface of the substrate or applying such a polymerizable composition onto the surface of a layer provided on the substrate and subjecting the applied polymerizable composition to a curing treatment, the photochromic layer can be formed as a cured layer containing one or more compounds represented by General Formula A and one or more compounds represented by General Formula B. As an application method, a known application method such as a spin coating method, a dip coating method, a spray coating method, an ink jet method, a nozzle coating method, and a slit coating method can be adopted. A curing treatment can be an irradiation with light and/or heat treatment. The polymerizable composition can further contain one or more additives (for example, a polymerization initiator or the like) in addition to one or more polymerizable compounds. As the polymerization reaction of the polymerizable compound proceeds, the polymerizable composition is cured, and thereby a cured layer can be formed.

The thickness of the photochromic layer can be 5 µm or more, 10 µm or more, or 20 µm or more, for example, and can be 80 µm or less, 70 µm or less, or 50 µm or less, for example.

### <Polymerizable Compound>

In the present invention and the present specification, a polymerizable compound refers to a compound having one or more polymerizable groups in one molecule, and the term "polymerizable group" refers to a reactive group capable of a polymerization reaction. Examples of polymerizable groups include an acryloyl group, a methacryloyl group, a vinyl group, a vinyl ether group, an epoxy group, a thiol group, an oxetane group, a hydroxy group, a carboxy group, an amino group, and an isocyanate group.

Examples of polymerizable compounds that can be used for forming the above-mentioned substrate and the above-mentioned photochromic layer include the following compounds.

### (Episulfide-Based Compound)

Episulfide-based compounds are compounds having two or more episulfide groups in one molecule. An episulfide group is a polymerizable group capable of ring-opening polymerization. Specific examples of episulfide-based compounds include bis(1,2-epithioethyl)sulfide, bis(1,2-epithioethyl)disulfide, bis(2,3-epithiopropyl)sulfide, bis(2,3-epithiopropylthio)methane, bis(2,3-epithiopropyl)disulfide, bis(2,3-epithiopropyldithio)methane, bis(2,3-epithiopropyldithio)ethane, bis(6,7-epithio-3,4-dithiaheptyl)sulfide, bis(6,7-epithio-3,4-dithiaheptyl)disulfide, 1,4-dithiane-2,5-bis(2,3-epithiopropyldithiomethyl), 1,3-bis(2,3-epithiopropyldithiomethyl)benzene, 1,6-bis(2,3-epithiopropyldithiomethyl)-2-(2,3-epithiopropyldithioethylthio)-4-thiahexane, 1,2,3-tris(2,3-epithiopropyldithio)propane, 1,1,1,1-tetrakis(2,3-epithiopropyldithiomethyl)methane, 1,3-bis(2,3-epithiopropyldithio)-2-thiapropane, 1,4-bis(2,3-epithiopropyldithio)-2,3-dithiabutane, 1,1,1-tris(2,3-epithiopropyldithio)methane, 1,1,1-tris(2,3-epithiopropyldithiomethylthio)methane, 1,1,2,2-tetrakis(2,3-epithiopropyldithio)ethane, 1,1,2,2-tetrakis(2,3-epithiopropyldithiomethylthio)ethane, 1,1,3,3-tetrakis(2,3-epithiopropyldithio)propane, 1,1,3,3-tetrakis(2,3-epithiopropyldithiomethylthio)propane, 2-[1,1-bis(2,3-epithiopropyldithio)methyl]-1,3-dithiethane, and 2-[1,1-bis(2,3-epithiopropyldithiomethylthio)methyl]-1,3-dithiethane.

### (Thietanyl-Based Compound)

A thietanyl-based compound is a thietane compound having two or more thietanyl groups in one molecule. A thietanyl group is a polymerizable group capable of ring-opening polymerization. Some thietanyl-based compounds have multiple thietanyl groups and also an episulfide group. Examples of such compounds are described in the above-mentioned examples of episulfide-based compounds. Other thietanyl-based compounds include metal-containing thietane compounds having metal atoms in the molecule and non-metallic thietane compounds containing no metal.

Specific examples of non-metallic thietane compounds include bis(3-thietanyl)disulfide, bis(3-thietanyl)sulfide, bis(3-thietanyl)trisulfide, bis(3-thietanyl)tetrasulfide, 1,4-bis(3-thietanyl)-1,3,4-trithiabutane, 1,5-bis(3-thietanyl)-1,2,4,5-tetrathiapentane, 1,6-bis(3-thietanyl)-1,3,4,6-tetrathiahexane, 1,6-bis(3-thietanyl)-1,3,5,6-tetrathiahexane, 1,7-bis(3-thietanyl)-1,2,4,5,7-pentathiaheptane, 1,7-bis(3-thietanylthio)-1,2,4,6,7-pentathiaheptane, 1,1-bis(3-thietanylthio)methane, 1,2-bis(3-thietanylthio)ethane, 1,2,3-tris(3-thietanylthio)propane, 1,8-bis(3-thietanylthio)-4-(3-thietanylthiomethyl)-3,6-dithiaoctane, 1,11-bis(3-thietanylthio)-4,8-bis(3-thietanylthiomethyl)-3,6,9-trithiaundecane, 1,11-bis(3-thietanylthio)-4,7-bis(3-thietanylthiomethyl)-3,6,9-trithiaundecane, 1,11-bis(3-thietanylthio)-5,7-bis(3-thietanylthiomethyl)-3,6,9-trithiaundecane, 2,5-bis(3-thietanylthiomethyl)-1,4-dithiane, 2,5-bis[[2-(3-thietanylthio)ethyl]thiomethyl]-1,4-dithiane, 2,5-bis(3-thietanylthiomethyl)-2,5-dimethyl-1,4-dithiane, bisthietanyl sulfide, bis(thietanylthio)methane, 3-[<(thietanylthio)methylthio>methylthio]thietane, bisthietanyl disulfide, bisthietanyl trisulfide, bisthietanyl tetrasulfide, bisthietanyl pentasulfide, 1,4-bis(3-thietanyldithio)-2,3-dithiabutane, 1,1,1-tris(3-thietanyldithio)methane, 1,1,1-tris(3-thietanyldithiomethylthio)methane, 1,1,2,2-tetrakis(3-thietanyldithio)ethane, and 1,1,2,2-tetrakis(3-thietanyldithiomethylthio) ethane.

Examples of metal-containing thietane compounds include compounds having, in the molecule as metal atoms, group 14 atoms such as Sn atoms, Si atoms, Ge atoms, and Pb atoms; group 4 elements such as Zr atoms and Ti atoms; group 13 atoms such as Al atoms; and group 12 atoms such as Zn atoms. Specific examples thereof include alkylthio(thietanylthio)tin, bis(alkylthio)bis(thietanylthio)tin, alkylthio(alkylthio)bis(thietanylthio)tin, bis(thietanylthio)cyclic dithiotin compounds, and alkyl(thietanylthio)tin compounds.

Specific examples of alkylthio(thietanylthio)tin include methylthiotris(thietanylthio)tin, ethylthiotris(thietanylthio)tin, propylthiotris(thietanylthio)tin, and isopropylthiotris(thietanylthio)tin.

Specific examples of bis(alkylthio)bis(thietanylthio)tin include bis(methylthio)bis(thietanylthio)tin, bis(ethylthio)bis(thietanylthio)tin, bis(propylthio)bis(thietanylthio)tin, and bis(isopropylthio)bis(thietanylthio)tin.

Specific examples of alkylthio(alkylthio)bis(thietanylthio)tin include ethylthio(methylthio)bis(thietanylthio)tin, methylthio(propylthio)bis(thietanylthio)tin, isopropylthio(methylthio)bis(thietanylthio)tin, ethylthio(propylthio)bis(thietanylthio)tin, ethylthio(isopropylthio)bis(thietanylthio)tin, and isopropylthio(propylthio)bis(thietanylthio)tin.

Specific examples of the bis(thietanylthio)cyclic dithiotin compounds include bis(thietanylthio)dithiastannetane, bis(thietanylthio)dithiastannolane, bis(thietanylthio)dithiastanninane, and bis(thietanylthio)trithiastannocane.

Specific examples of alkyl(thietanylthio)tin compounds include methyltris(thietanylthio)tin, dimethylbis(thietanylthio)tin, butyltris(thietanylthio)tin, tetrakis(thietanylthio)tin, tetrakis(thietanylthio)germanium, and tris(thietanylthio)bismuth.

### (Polyamine Compound)

A polyamine compound is a compound having two or more NH₂ groups in one molecule, and can form a urea bond by reaction with polyisocyanate and can form a thiourea bond by reaction with polyisothiocyanate. Specific examples of polyamine compounds include ethylenediamine, hexamethylenediamine, isophoronediamine, nonamethylenediamine, undecamethylenediamine, dodecamethylenediamine, meta-xylenediamine, 1,3-propanediamine, putrescine, 2-(2-aminoethylamino)ethanol, diethylenetriamine, p-phenylenediamine, m-phenylenediamine, melamine, and 1,3,5-benzenetriamine.

### (Epoxy-Based Compound)

Epoxy-based compounds are compounds having an epoxy group in the molecule. An epoxy group is a polymerizable group capable of ring-opening polymerization. Epoxy compounds are generally classified into aliphatic epoxy compounds, alicyclic epoxy compounds, and aromatic epoxy compounds.

Specific examples of aliphatic epoxy compounds include ethylene oxide, 2-ethyloxirane, butyl glycidyl ether, phenyl glycidyl ether, 2,2'-methylenebisoxirane, 1,6-hexanediol diglycidyl ether, ethylene glycol diglycidyl ether, diethylene glycol diglycidyl ether, triethylene glycol diglycidyl ether, tetraethylene glycol diglycidyl ether, nonaethylene glycol diglycidyl ether, propylene glycol diglycidyl ether, dipropylene glycol diglycidyl ether, tripropylene glycol diglycidyl ether, tetrapropylene glycol diglycidyl ether, nonapropylene glycol diglycidyl ether, neopentyl glycol diglycidyl ether, trimethylolpropane triglycidyl ether, glycerol triglycidyl ether, diglycerol tetraglycidyl ether, pentaerythritol tetraglycidyl ether, and triglycidyl ether of tris(2-hydroxyethyl)isocyanurate.

Specific examples of alicyclic epoxy compounds include isophorone diol diglycidyl ether and bis-2,2-hydroxycyclohexylpropane diglycidyl ether.

Specific examples of aromatic epoxy compounds include resorcinol diglycidyl ether, bisphenol A diglycidyl ether, bisphenol F diglycidyl ether, bisphenol S diglycidyl ether, orthophthalic acid diglycidyl ester, phenol novolac polyglycidyl ether, and cresol novolac polyglycidyl ether.

In addition to the above-mentioned examples, an epoxy-based compound having a sulfur atom in the molecule can also be used together with the epoxy group. Such epoxy-based compounds containing a sulfur atom include linear aliphatic compounds and cycloaliphatic compounds.

Specific examples of linear aliphatic epoxy-based compounds containing a sulfur atom include bis(2,3-epoxypropyl)sulfide, bis(2,3-epoxypropyl)disulfide, bis(2,3-epoxypropylthio)methane, 1,2-bis(2,3-epoxypropylthio)ethane, 1,2-bis(2,3-epoxypropylthio)propane, 1,3-bis(2,3-epoxypropylthio)propane, 1,3-bis(2,3-epoxypropylthio)-2-methylpropane, 1,4-bis(2,3-epoxypropylthio)butane, 1,4-bis(2,3-epoxypropylthio)-2-methylbutane, 1,3-bis(2,3-epoxypropylthio)butane, 1,5-bis(2,3-epoxypropylthio)pentane, 1,5-bis(2,3-epoxypropylthio)-2-methylpentane, 1,5-bis(2,3-epoxypropylthio)-3-thiapentane, 1,6-bis(2,3-epoxypropylthio)hexane, 1,6-bis(2,3-epoxypropylthio)-2-methylhexane, 3,8-bis(2,3-epoxypropylthio)-3,6-dithiaoctane, 1,2,3-tris(2,3-epoxypropylthio)propane, 2,2-bis(2,3-epoxypropylthio)-1,3-bis(2,3-epoxypropylthiomethyl)propane, and 2,2-bis(2,3-epoxypropylthiomethyl)-1-(2,3-epoxypropylthio)butane.

Specific examples of cycloaliphatic epoxy-based compounds containing a sulfur atom include 1,3-bis(2,3-epoxypropylthio)cyclohexane, 1,4-bis(2,3-epoxypropylthio)cyclohexane, 1,3-bis(2,3-epoxypropylthiomethyl)cyclohexane, 1,4-bis(2,3-epoxypropylthiomethyl)cyclohexane, 2,5-bis(2,3-epoxypropylthiomethyl)-1,4-dithiane, 2,5-bis[<2-(2,3-epoxypropylthio)ethyl>thiomethyl]-1,4-dithiane, and 2,5-bis(2,3-epoxypropylthiomethyl)-2,5-dimethyl-1,4-dithiane.

### (Compound Having Radically Polymerizable Group)

A compound having a radically polymerizable group is a polymerizable group capable of radical polymerization. Examples of radically polymerizable groups include an acryloyl group, a methacryloyl group, an allyl group, and a vinyl group.

A compound having a polymerizable group selected from the group consisting of an acryloyl group and a methacryloyl group is hereinafter referred to as a "(meth)acrylate compound". Specific examples of (meth)acrylate compounds include ethylene glycol di(meth)acrylate, diethylene glycol di(meth)acrylate, triethylene glycol di(meth)acrylate, tetraethylene glycol di(meth)acrylate, polyethylene glycol di(meth)acrylate, propylene glycol di(meth)acrylate, dipropylene glycol di(meth)acrylate, tripropylene glycol di(meth)acrylate, polypropylene glycol di(meth)acrylate, neopentyl glycol di(meth)acrylate, ethylene glycol bisglycidyl (meth)acrylate, bisphenol A di(meth)acrylate, 2,2-bis(4-(meth)acryloxyethoxyphenyl)propane, 2,2-bis(4-(meth)acroxydiethoxyphenyl)propane, 2,2-bis(4-(meth)acryloyloxyethoxyphenyl)propane, 2,2-bis(3,5-dibromo-4-(meth)acryloyloxyethoxyphenyl)propane, 2,2-bis(4-(meth)acryloyloxydipropoxyphenyl)propane, bisphenol F di(meth) acrylate, 1,1-bis(4-(meth)acryloxyethoxyphenyl)methane, 1,1-bis(4-(meth)acroxydiethoxyphenyl)methane, dimethyloltricyclodecane di(meth)acrylate, trimethylolpropane tri(meth)acrylate, ditrimethylolpropane tetra(meth)acrylate, glycerol di(meth)acrylate, pentaerythritol tri(meth)acrylate, pentaerythritol tetra(meth)acrylate, methylthio(meth)acrylate, phenylthio(meth)acrylate, benzylthio(meth)acrylate, xylylenedithiol di(meth)acrylate, mercaptoethyl sulfide di(meth)acrylate, and bifunctional urethane (meth)acrylate.

Specific examples of compounds (allyl compounds) having an allyl group include allyl glycidyl ether, diallyl phthalate, diallyl terephthalate, diallyl isophthalate, diallyl carbonate, diethylene glycol bisallyl carbonate, methoxypolyethylene glycol allyl ether, polyethylene glycol allyl ether, methoxypolyethylene glycol-polypropylene glycol allyl ether, butoxy polyethylene glycol-polypropylene glycol allyl ether, methacryloyloxy polyethylene glycol-polypropylene glycol allyl ether, phenoxy polyethylene glycol allyl ether, and methacryloyloxy polyethylene glycol allyl ether.

Examples of compounds (vinyl compounds) having a vinyl group include α-methylstyrene, α-methylstyrene dimer, styrene, chlorostyrene, methylstyrene, bromostyrene, dibromostyrene, divinylbenzene, and 3,9-divinylspirobi(m-dioxane).

The above-mentioned photochromic article can include, at an arbitrary location, one or more layers known as functional layers of the photochromic article such as a protective layer for improving the durability of the photochromic article, an antireflection layer, a water-repellent or hydrophilic antifouling layer, an antifogging layer, and a primer layer for improving adhesiveness between layers.

The above-mentioned photochromic article can be an optical article. One aspect of the optical article is spectacle lenses. Such spectacle lenses can also be called photochromic lenses or photochromic spectacle lenses. In addition, as one aspect of the optical article, there are a lens for goggles, a visor (brim) portion of a sun visor, a shield member of a helmet, and the like. An optical article having an antiglare function can be obtained by applying the above-mentioned photochromic composition, which is a polymerizable composition, onto a substrate for these optical articles, and subjecting the applied composition to a curing treatment to form a photochromic layer.

### [Spectacles]

One aspect of the present invention relates to spectacles having a spectacle lens which is one aspect of the above-mentioned photochromic article. The details of the spectacle lens included in these spectacles is as described above. By providing such a spectacle lens, the above-mentioned spectacles can exhibit an antiglare effect as in the case of sunglasses by coloring of the photochromic compound upon irradiate with sunlight outdoors, and transmittance can be restored by fading of the photochromic compound when returned indoors. For the above-mentioned spectacles, a known technique can be applied to the configuration of a frame and the like.

### [Examples]

Hereinbelow, the present invention will be further described with reference to examples. However, the present invention is not limited to the embodiments described in the examples. The symbol "%" described below indicates mass%.

### [Synthesis of Compounds]

Compounds 1 to 15 shown below were synthesized by referring to the references described above regarding the synthesis method of the compounds. The compounds were identified in the same manner as described in the reference publication to confirm whether the compounds shown below were synthesized. Compounds 1 to 7 are compounds represented by General Formula A, and compounds 8 to 15 are compounds represented by General Formula B.

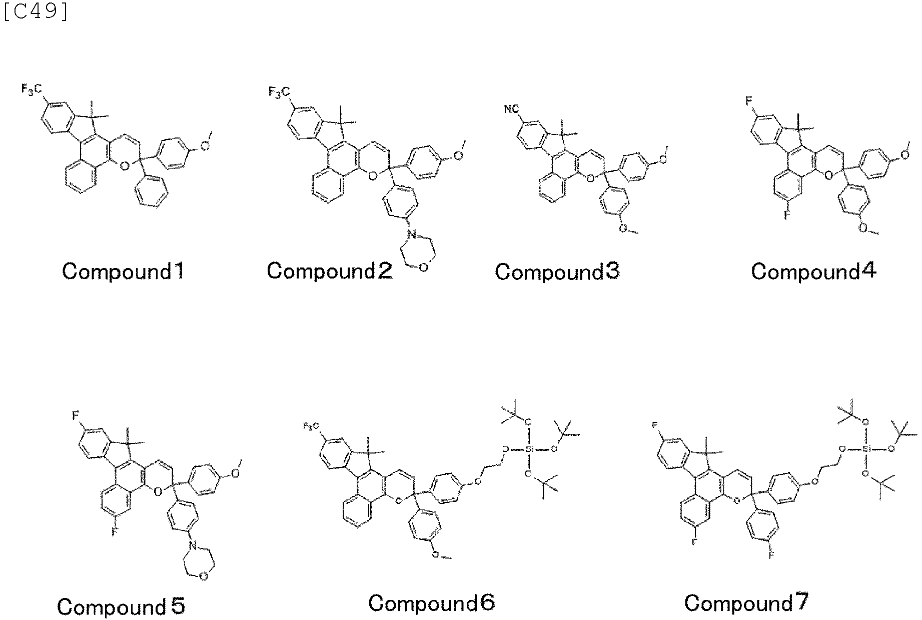

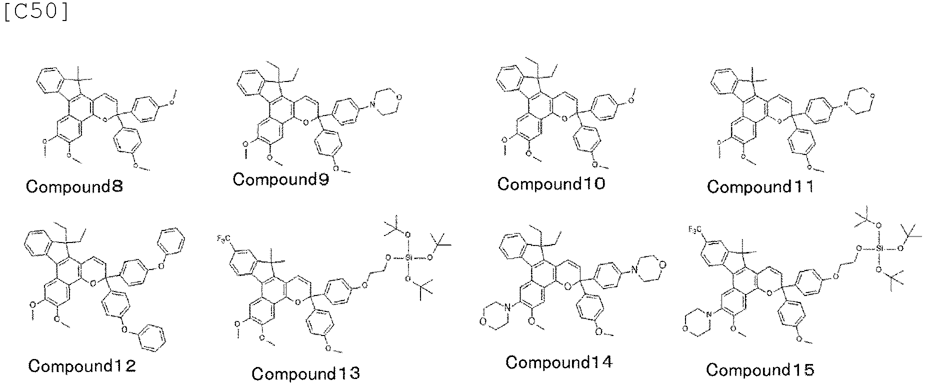

### [Examples 1 to 28 and Comparative Examples 1 to 3]

### <Preparation of Photochromic Composition (Polymerizable Composition)>

In a plastic container, with respect to a total of 100 parts by mass of (meth)acrylate, 68 parts by mass of polyethylene glycol diacrylate, 12 parts by mass of trimethylolpropane trimethacrylate, and 20 parts by mass of neopentyl glycol dimethacrylate were mixed to prepare a (meth)acrylate mixture. A photochromic compound was mixed to 100 parts by mass of this (meth)acrylate mixture such that the amount was 2.5 parts by mass. For compositions containing multiple photochromic compounds, Table 2 shows the mass ratio of each photochromic compound when the total amount of photochromic compounds was 10. Furthermore, a photopolymerization initiator (phenylbis(2,4,6-trimethylbenzoyl)phosphine oxide), an antioxidant [bis(3-tert-butyl-4-hydroxy-5-methylphenyl)propionic acid)] [ethylene bis(oxyethylene), and a light stabilizer (bis(1,2,2,6,6-pentamethyl-4-piperidyl)sebacic acid) were mixed, and after sufficient stirring, a silane coupling agent (γ-methacryloxypropyltrimethoxysilane) was added dropwise while stirring. Thereafter, defoaming was caused by an automatic revolution type stirring and defoaming device.

A photochromic composition was prepared by the method described above.

A comparative compound used in Comparative Example 3 was the following compound.

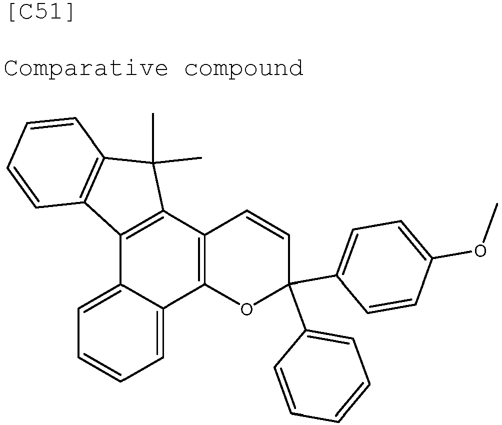

### <Formation of Primer Layer>

A plastic lens substrate (manufactured by HOYA CORPORATION, trade name: EYAS, center thickness: 2.5 mm, diameter: 75 mm, spherical lens power: -4.00) was immersed in an aqueous sodium hydroxide solution having a concentration of 10 mass% (liquid temperature 60°C) for 5 minutes to perform alkaline cleaning, further cleaning with pure water was performed, and drying was performed. Thereafter, in an environment of room temperature and relative humidity of 40% to 60% and by a spin coating method, an aqueous polyurethane resin liquid (polycarbonate polyol-based polyurethane emulsion, viscosity: 100 cPs, concentration of solid contents: 38 mass%) was applied to the convex surface of this plastic lens substrate at a rotation speed of 1500 rpm for 1 minute using a spin coater MS-B150 manufactured by Mikasa Corporation, and thereafter air drying was performed for 15 minutes to form a primer layer having a thickness of 5.5 µm.

### <Formation of Photochromic Layer>

The photochromic composition prepared as above was added dropwise onto the above-mentioned primer layer, and was applied by a spin coating method using a program to change the rotation speed from 500 rpm to 1500 rpm over 1 minute in slope mode and to further cause rotation at 1500 rpm for 5 seconds using MS-B150 manufactured by Mikasa Corporation. Thereafter, the photochromic composition applied onto the primer layer formed on the plastic lens substrate was irradiated with ultraviolet rays (main wavelength: 405 nm) for 40 seconds in a nitrogen atmosphere (oxygen concentration: 500 ppm or less), and this composition was cured to form a photochromic layer. The thickness of the formed photochromic layer was 45 µm.

Thereby, a photochromic article (spectacle lens) was produced.

### [Evaluation Method]

### <Evaluation of Coloring Density>

The luminous reflectance was obtained by the following method in accordance with JIS T 7333:2005.

The convex surface of each spectacle lens of the examples and the comparative examples was irradiated with light through an aeromass filter for 15 minutes using a xenon lamp as a light source to cause coloring of the photochromic layer. This irradiation with light was performed such that the irradiance and the tolerance of the irradiance were the values shown in Table 1 as defined in JIS T 7333:2005. The transmittance when coloring was measured with a spectrophotometer manufactured by Otsuka Electronics Co., Ltd. Table 2 shows the luminous transmittance T (%) obtained from the measurement results in the wavelength range of 380 nm to 780 nm. A smaller value of T (%) means that the photochromic compound developed color at a higher density.

**[Table 1]**

| Wavelength region (nm) | Irradiance (W/m²) | Tolerance of irradiance (W/m²) |
|---|---|---|
| 300 to 340 | < 2.5 | - |
| 340 to 380 | 5.6 | ± 1.5 |
| 380 to 420 | 12 | ± 3.0 |
| 420 to 460 | 12 | ± 3.0 |
| 460 to 500 | 26 | ± 2.6 |

### <Evaluation of Fading Speed>

The fading speed was evaluated by the following method.

The transmittance (measurement wavelength: 550 nm) of each spectacle lens of the examples and the comparative examples before irradiation with light (uncolored state) was measured with a spectrophotometer manufactured by Otsuka Electronics Co., Ltd. The transmittance measured herein is called "initial transmittance".

Each of the spectacle lenses was irradiated with light through an aeromass filter for 15 minutes using a xenon lamp as a light source to cause coloring of the photochromic layer. This irradiation with light was performed such that the irradiance and the tolerance of the irradiance were the values shown in Table 1 as defined in JIS T 7333:2005. The transmittance when coloring was measured in the same manner as the initial transmittance. The transmittance measured herein is called "transmittance when coloring".

Thereafter, the time required for the transmittance to reach [(initial transmittance - transmittance when coloring)/2] from the time when irradiation with light was stopped was measured. This time is called "half-life time". It can be said that the shorter the half-life time, the faster the fading speed. Table 2 shows the obtained half-life time.

**[Table 2]**

| | Photochromic compound | Mass ratio | Coloring density T (%) | Fading speed half-life time (sec) |
|---|---|---|---|---|
| Example 1 | Compound 1/Compound 8 | 1/9 | 18.6 | 190 |
| Example 2 | Compound 1/Compound 8/Compound 9 | 1/5/4 | 18.2 | 160 |
| Example 3 | Compound 1/Compound 9/Compound 10 | 1/5/4 | 19.5 | 140 |
| Example 4 | Compound 1/Compound 10/Compound 14 | 1/5/4 | 18.4 | 145 |
| Example 5 | Compound 2/Compound 10 | 1/9 | 19.2 | 155 |
| Example 6 | Compound 2/Compound 10/Compound 11 | 1/5/4 | 17.8 | 170 |
| Example 7 | Compound 2/Compound 8/Compound 9 | 1/5/4 | 18.3 | 165 |
| Example 8 | Compound 2/Compound 12/Compound 15 | 1/5/4 | 19.0 | 170 |
| Example 9 | Compound 3/Compound 13/Compound 15 | 1/5/4 | 18.6 | 145 |
| Example 10 | Compound 3/Compound 11/Compound 12 | 1/5/4 | 18.7 | 185 |
| Example 11 | Compound 2/Compound 3/Compound 14 | 1/1/8 | 18.0 | 140 |
| Example 12 | Compound 3/Compound 11 | 1/9 | 18.2 | 190 |
| Example 13 | Compound 4/Compound 13/Compound 15 | 1/5/4 | 18.0 | 140 |
| Example 14 | Compound 4/Compound 11/Compound 12 | 1/5/4 | 17.8 | 170 |
| Example 15 | Compound 4/Compound 14 | 1/9 | 18.1 | 135 |
| Example 16 | Compound 4/Compound 11 | 1/9 | 17.8 | 165 |
| Example 17 | Compound 5/Compound 13/Compound 15 | 1/5/4 | 18.2 | 150 |
| Example 18 | Compound 5/Compound 11/Compound 12 | 1/5/4 | 17.7 | 155 |
| Example 19 | Compound 5/Compound 14 | 1/9 | 17.8 | 145 |
| Example 20 | Compound 3/Compound 5/Compound 11 | 1/1/8 | 17.4 | 185 |
| Example 21 | Compound 6/Compound 13 | 1/9 | 19.0 | 130 |
| Example 22 | Compound 6/Compound 13/Compound 15 | 1/5/4 | 19.8 | 125 |
| Example 23 | Compound 6/Compound 9/Compound 10 | 1/5/4 | 19.2 | 135 |
| Example 24 | Compound 6/Compound 9/Compound 12 | 1/5/4 | 19.0 | 145 |
| Example 25 | Compound 7/Compound 13 | 1/9 | 18.8 | 130 |
| Example 26 | Compound 7/Compound 13/Compound 15 | 1/5/4 | 19.2 | 125 |
| Example 27 | Compound 7/Compound 9/Compound 10 | 1/5/4 | 18.6 | 140 |
| Example 28 | Compound 7/Compound 9/Compound 12 | 1/5/4 | 18.8 | 145 |
| Comparative Example 1 | Compound 1 | 10 | 25.0 | 150 |
| Comparative Example 2 | Compound 8 | 10 | 21.0 | 240 |
| Comparative Example 3 | Comparative compound/Compound 8/Compound 11 | 1/5/4 | 17.8 | 280 |

From the results shown in Table 2, it could be confirmed that by combining the compound represented by General Formula A and the compound represented by General Formula B, a photochromic article that can color at a high density in the visible range and exhibits a fast fading speed can be provided.

Finally, each of aspects described above will be summarized.

According to one aspect, a photochromic article and a photochromic composition which contain one or more compounds represented by General Formula A and one or more compounds represented by General Formula B are provided.

In one aspect, R¹ and R² in General Formula A may each independently represent a substituted or unsubstituted alkyl group having 1 to 20 carbon atoms.

In one aspect, R¹ and R² in General Formula A may each independently represent a methyl group, an ethyl group, a propyl group, a butyl group, a pentyl group, or a hexyl group.

In one aspect, B¹ and B² in General Formula A and B³ and B⁴ in General Formula B may each independently represent a substituted or unsubstituted phenyl group. When the phenyl group has multiple substituents, these substituents may be bonded to form a ring.

In one aspect, an electron-withdrawing group in General Formula A may be a halogen atom, a perfluoroalkyl group having 1 to 6 carbon atoms, a perfluorophenyl group, a perfluoroalkylphenyl group, or a cyano group.

In one aspect, the above-mentioned halogen atom may be a fluorine atom.

In one aspect, the above-mentioned perfluoroalkyl group may be a trifluoromethyl group.

In one aspect, one or more selected from the group consisting of R¹, R², B¹, and B² in General Formula A and R⁷ to R¹², B³, and B⁴ in General Formula B represent a substituent, and such a substituent may be the following substituent:
a substituent R^{m} selected from the group consisting of a hydroxy group, a linear or branched alkyl group having 1 to 18 carbon atoms, a cycloaliphatic alkyl group of a single-ring type or a multi-ring type such as a bicyclic ring which has 5 to 18 carbon atoms, a linear or branched alkoxy group having 1 to 24 constituent atoms, a non-aromatic cyclic substituent having 1 to 24 constituent atoms, a linear or branched perfluoroalkyl group having 1 to 18 carbon atoms, a linear or branched perfluoroalkoxy group, a linear or branched alkylsulfide group having 1 to 24 constituent atoms, an aryl group, an aryloxy group, an arylsulfide group, a heteroaryl group, an amino group, a monoalkylamino group, a dialkylamino group, a monoarylamino group, a diarylamino group, a cyclic amino group such as a piperidino group, a morpholino group, a thiomorpholino group, a tetrahydroquinolino group, and a tetrahydroisoquinolino group, an ethynyl group, a mercapto group, a silyl group, a sulfonic acid group, an alkylsulfonyl group, a formyl group, a carboxy group, a cyano group, and a halogen atom;
a substituent in which R^{m} is further substituted with one or more same or different R^{m}' s; or
a solubilizing group.

In one aspect, R⁷ and R⁸ in General Formula B may each independently represent a substituted or unsubstituted alkyl group having 1 to 20 carbon atoms.

In one aspect, R⁷ and R⁸ in General Formula B may each independently represent a methyl group, an ethyl group, a propyl group, a butyl group, a pentyl group, or a hexyl group.

In one aspect, in General Formula B, R¹³ and R¹⁴ may each independently represent an electron-donating group selected from the group consisting of a methoxy group, an ethoxy group, a phenoxy group, a methylsulfide group, a phenylsulfide group, a dimethylamino group, a pyrrolidino group, a piperidino group, a morpholino group, and a thiomorpholino group.

In one aspect, in the above-mentioned photochromic article and the above-mentioned photochromic composition, the compound represented by General Formula B may be more than the compound represented by General Formula A on a mass basis.

In one aspect, the above-mentioned photochromic article and the above-mentioned photochromic composition may contain one type of the compound represented by General Formula A, and two types of the compound represented by General Formula B.

In one aspect, the above-mentioned photochromic article may be a photochromic article which has a substrate and a photochromic, and which contains, in this photochromic layer, one or more compounds represented by General Formula A and one or more compounds represented by General Formula B.

In one aspect, the above-mentioned photochromic layer may be a cured layer obtained by curing a polymerizable composition.

In one aspect, the above-mentioned photochromic composition may contain a polymerizable compound.

In one aspect, the above-mentioned photochromic article may be a spectacle lens.

In one aspect, the above-mentioned photochromic article may be a lens for goggles.

In one aspect, the above-mentioned photochromic article may be a visor portion of a sun visor.

In one aspect, the above-mentioned photochromic article may be a shield member of a helmet.

According to one aspect, spectacles including the above-mentioned spectacle lens are provided.

It should be considered that the embodiments described this time are examples in all respects and not restrictive. The scope of the present invention is indicated by the scope of the claims rather than the above description.

### [Industrial Applicability]

One aspect of the present invention is useful in technical fields such as spectacles, goggles, sun visors, and helmets.

## Claims

1. A composition, which is a photochromic composition comprising:
at least one compound of formula (A): 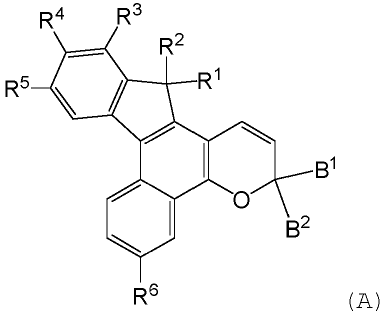
wherein R¹, R², B¹ and B² each independently are H or a substituent, and R³-R⁶ each independently are H or an electron-withdrawing group, provided that at least one of one of R³-R⁶ is an electron-withdrawing group, and
at least one compound of formula (B): 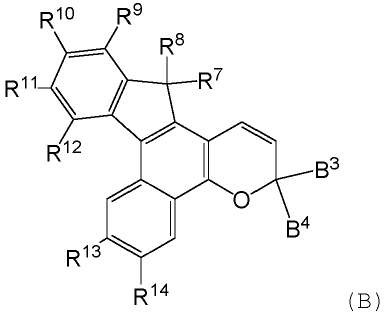
wherein R⁷-R¹², B³ and B⁴ each independently are H or a substituent, and R¹³ and R¹⁴ each independently are H or an electron-donating group, provided that at least one of R¹³ and R¹⁴ is an electron-donating group.

2. The composition of claim 1, wherein R¹ and R² in formula (A) each independently are optionally substituted C₁₋₂₀-alkyl, and preferably methyl, ethyl, propyl, butyl, pentyl or hexyl.

3. The composition of claim 1 or 2, wherein B¹ and B² in formula (A) and B³ and B⁴ in formula (B) each independently are optionally substituted phenyl, wherein a plurality of substituents may be bonded to form a ring.

4. The composition of any of claims 1-3, wherein the electron-withdrawing group is halogen, C₁₋₆-perfluoroalkyl, perfluorophenyl, perfluoroalkylphenyl or cyano.

5. The composition of claim 4, wherein the halogen is F.

6. The composition of 4, wherein the perfluoroalkyl group is trifluoromethyl.

7. The composition of any of claims 1-6, wherein at least one of R¹, R², B¹ and B² in formula (A) and R⁷-R¹², B³ and B⁴ in formula (B) is
- a group R^{m} selected from hydroxy, linear or branched C₁₋₁₈-alkyl, a single- or multi-ring type cycloaliphatic alkyl group such as a C₅₋₁₈-bicyclic ring, linear or branched C₁₋₂₄-alkoxy, a non-aromatic cyclic C₁₋₂₄-substituent, linear or branched C₁₋₁₈-perfluoroalkyl, linear or branched perfluoroalkoxy, linear or branched C₁₋₂₄-alkylsulfide, aryl, aryloxy, arylsulfide, heteroaryl, amino, monoalkylamino, dialkylamino, monoarylamino, diarylamino, cyclic amino such as piperidino, morpholino, thiomorpholino, tetrahydroquinolino, tetrahydroisoquinolino, ethynyl, mercapto, silyl, sulfonic acid, alkylsulfonyl, formyl, carboxy, cyano and halogen;
- a group wherein R^{m} is further substituted with one or more groups each individually selected from R^{m}; or
- a solubilizing group.

8. The composition of any of claims 1-7, wherein R⁷ and R⁸ in formula (B) each independently are optionally substituted C₁₋₂₀-alkyl, and preferably methyl, ethyl, propyl, butyl, pentyl or hexyl.

9. The composition of any of claims 1-8, wherein R¹³ and R¹⁴ in formula (B) each independently are an electron-donating group selected from methoxy, ethoxy, phenoxy, methylsulfide, phenylsulfide, dimethylamino, pyrrolidino, piperidino, morpholino and thiomorpholino.

10. The composition of any of claims 1-9, wherein the compound of formula (B) is more than the compound of formula (A) on a mass basis.

11. The composition of any of claims 1-10, further comprising a polymerizable compound.

12. A photochromic article comprising a cured product obtained by curing the composition of claim 11.

13. The photochromic article of claim 12, comprising a substrate; and a photochromic layer which is the cured product.

14. The photochromic article of claim 12 or 13, which is a spectacle lens; a lens for goggles, a visor portion of a sun visor, or a shield member of a helmet.

15. Spectacles comprising the spectacle lens of claim 14.

## Patentansprüche

1. Zusammensetzung, die eine photochrome Zusammensetzung ist, umfassend:
mindestens eine Verbindung der Formel (A): 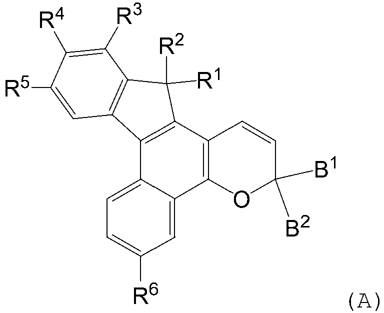
worin R¹, R², B¹ und B² jeweils unabhängig voneinander H oder ein Substituent sind und R³-R⁶ jeweils unabhängig voneinander H oder eine elektronenziehende Gruppe sind, mit der Maßgabe, dass mindestens eines von R³-R⁶ eine elektronenziehende Gruppe ist, und
mindestens eine Verbindung der Formel (B): 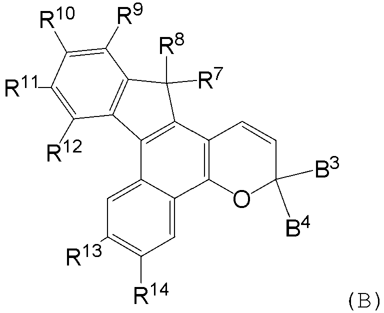
worin R⁷-R¹², B³ und B⁴ jeweils unabhängig voneinander H oder ein Substituent sind und R¹³ und R¹⁴ jeweils unabhängig voneinander H oder eine elektronenschiebende Gruppe sind, mit der Maßgabe, dass mindestens eines von R¹³ und R¹⁴ eine elektronschiebende Gruppe ist.

2. Zusammensetzung gemäß Anspruch 1, wobei R¹ und R² in Formel (A) jeweils unabhängig voneinander optional substituiertes C₁₋₂₀-Alkyl und bevorzugt Methyl, Ethyl, Propyl, Butyl, Pentyl oder Hexyl sind.

3. Zusammensetzung gemäß Anspruch 1 oder 2, wobei B¹ und B² in Formel (A) und B³ und B⁴ in Formel (B) jeweils unabhängig voneinander optional substituiertes Phenyl sind, wobei mehrere Substituenten unter Bildung eines Rings verbunden sein können.

4. Zusammensetzung gemäß einem der Ansprüche 1-3, wobei die elektronenziehende Gruppe Halogen, C₁₋₆-Perfluoralkyl, Perfluorphenyl, Perfluoralkylphenyl oder Cyano ist.

5. Zusammensetzung gemäß Anspruch 4, wobei das Halogen F ist.

6. Zusammensetzung gemäß 4, wobei die Perfluoralkylgruppe Trifluormethyl ist.

7. Zusammensetzung gemäß einem der Ansprüche 1-6, wobei mindestens eines von R¹, R², B¹ und B² in Formel (A) und R⁷-R¹², B³ und B⁴ in Formel (B)
- eine Gruppe R^{m}, ausgewählt aus Hydroxy, linearem oder verzweigtem C₁₋₁₈-Alkyl, einer ein- oder mehrringigen cycloaliphatischen Alkylgruppe, beispielsweise ein C₅₋₁₈-bicyclischer Ring, linearem oder verzweigtem C₁₋₂₄-Alkoxy, einem nichtaromatischen cyclischen C₁₋₂₄-Substituenten, linearem oder verzweigtem C₁₋₁₈-Perfluoralkyl, linearem oder verzweigtem Perfluoralkoxy, linearem oder verzweigtem C₁₋₂₄-Alkylsulfid, Aryl, Aryloxy, Arylsulfid, Heteroaryl, Amino, Monoalkylamino, Dialkylamino, Monoarylamino, Diarylamino, cyclischem Amino, beispielsweise Piperidino, Morpholino, Thiomorpholino, Tetrahydrochinolino, Tetrahydroisochinolino, Ethinyl, Mercapto, Silyl, Sulfonsäure, Alkylsulfonyl, Formyl, Carboxy, Cyano und Halogen;
- eine Gruppe, in der R^{m} zusätzlich mit einer oder mehreren Gruppen substituiert ist, die jeweils individuell aus R^{m} ausgewählt sind; oder
- eine solubilisierende Gruppe
ist.

8. Zusammensetzung gemäß einem der Ansprüche 1-7, wobei R⁷ und R⁸ in Formel (B) jeweils unabhängig voneinander optional substituiertes C₁₋₂₀-Alkyl und bevorzugt Methyl, Ethyl, Propyl, Butyl, Pentyl oder Hexyl sind.

9. Zusammensetzung gemäß einem der Ansprüche 1-8, wobei R¹³ und R¹⁴ in Formel (B) jeweils unabhängig voneinander eine elektronenschiebende Gruppe, ausgewählt aus Methoxy, Ethoxy, Phenoxy, Methylsulfid, Phenylsulfid, Dimethylamino, Pyrrolidino, Piperidino, Morpholino und Thiomorpholino, sind.

10. Zusammensetzung gemäß einem der Ansprüche 1-9, wobei die Verbindung der Formel (B) auf Massenbasis mehr als die Verbindung der Formel (A) ausmacht.

11. Zusammensetzung gemäß einem der Ansprüche 1-10, ferner umfassend eine polymerisierbare Verbindung.

12. Photochromer Gegenstand, umfassend ein gehärtetes Produkt, erhalten durch Härten der Zusammensetzung gemäß Anspruch 11.

13. Photochromer Gegenstand gemäß Anspruch 12, umfassend ein Substrat; und eine photochrome Schicht, die das gehärtete Produkt ist.

14. Photochromer Gegenstand gemäß Anspruch 12 oder 13, der eine Brillenglaslinse, eine Linse für eine Schutzbrille, ein Visierbereich einer Sonnenblende oder ein Schildelement eines Helms ist.

15. Brille, umfassend die Brillenglaslinse gemäß Anspruch 14.

## Revendications

1. Composition, qui est une composition photochromique comprenant :
au moins un composé de formule (A) : 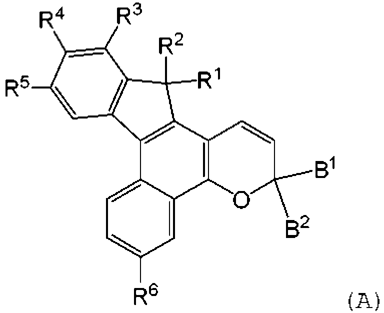
dans laquelle R¹, R², B¹ et B² sont chacun indépendamment H ou un substituant, et R³ à R⁶ sont chacun indépendamment H ou un groupe attracteur d'électrons, à condition qu'au moins l'un des R³ à R⁶ soit un groupe attracteur d'électrons, et
au moins un composé de formule (B) : 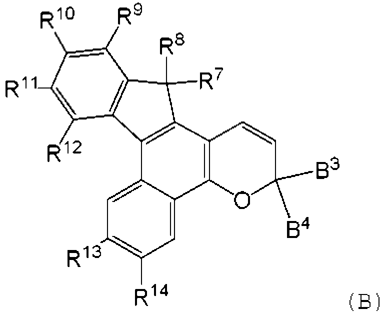
dans laquelle R⁷ à R¹², B³ et B⁴ sont chacun indépendamment H ou un substituant, et R¹³ et R¹⁴ sont chacun indépendamment H ou un groupe donneur d'électrons, à condition qu'au moins l'un des R¹³ et R¹⁴ soit un groupe donneur d'électrons.

2. Composition selon la revendication 1, dans laquelle R¹ et R² dans la formule (A) sont chacun indépendamment un alkyle en C₁₋₂₀ facultativement substitué, et de préférence un méthyle, un éthyle, un propyle, un butyle, un pentyle ou un hexyle.

3. Composition selon la revendication 1 ou la revendication 2, dans laquelle B¹ et B² dans la formule (A) et B³ et B⁴ dans la formule (B) sont chacun indépendamment un phényle facultativement substitué, dans laquelle une pluralité de substituants peuvent être liés pour former un cycle.

4. Composition selon l'une quelconque des revendications 1 à 3, dans laquelle le groupe attracteur d'électrons est un halogène, un perfluoroalkyle en C₁₋₆, un perfluorophényle, un perfluoroalkylphényle ou un cyano.

5. Composition selon la revendication 4, dans laquelle l'halogène est F.

6. Composition selon la revendication 4, dans laquelle le groupe perfluoroalkyle est un trifluorométhyle.

7. Composition selon l'une quelconque des revendications 1 à 6, dans laquelle au moins l'un des R¹, R², B¹ et B² dans la formule (A) et des R⁷ à R¹², B³ et B⁴ dans la formule (B) est
- un groupe R^{m} sélectionné parmi un hydroxy, un alkyle linéaire ou ramifié en C₁₋₁₈, un groupe alkyle cycloaliphatique de type mono- ou poly-cyclique tel qu'un cycle bicyclique en C₅ à C₁₈, un alcoxy linéaire ou ramifié en C₁₋₂₄, un substituant cyclique non aromatique en C_{4 -24}, un perfluoroalkyle linéaire ou ramifié en C₁₋₁₈, un perfluoroalcoxy linéaire ou ramifié, un alkylsulfure linéaire ou ramifié en C₁₋₂₄, un aryle, un aryloxy, un arylsulfure, un hétéroaryle, un amino, un monoalkylamino, un dialkylamino, un monoarylamino, un diarylamino, un amino cyclique tel que la pipéridine, un morpholino, un thiomorpholino, un tétrahydroquinoléino, un tétrahydroisoquinoléino, un éthynyle, un mercapto, un silyle, un acide sulfonique, un alkylsulfonyle, un formyle, un carboxyle, un cyano et un halogène ;
- un groupe dans lequel R^{m} est en outre substitué avec un ou plusieurs groupes sélectionnés chacun individuellement parmi R^{m} ; ou
- un groupe solubilisant.

8. Composition selon l'une quelconque des revendications 1 à 7, dans laquelle R⁷ et R⁸ dans la formule (B) sont chacun indépendamment un alkyle en C₁₋₂₀ facultativement substitué, et de préférence un méthyle, un éthyle, un propyle, un butyle, un pentyle ou un hexyle.

9. Composition selon l'une quelconque des revendications 1 à 8, dans laquelle R¹³ et R¹⁴ dans la formule (B) sont chacun indépendamment un groupe donneur d'électrons sélectionné parmi un méthoxy, un éthoxy, un phénoxy, un méthylsulfure, un phénylsulfure, un diméthylamino, une pyrrolidine, une pipéridine, un morpholino et un thiomorpholino.

10. Composition selon l'une quelconque des revendications 1 à 9, dans laquelle le composé de formule (B) présente une masse plus élevée que celle du composé de formule (A).

11. Composition selon l'une quelconque des revendications 1 à 10, comprenant en outre un composé polymérisable.

12. Article photochromique comprenant un produit durci obtenu par durcissement de la composition selon la revendication 11.

13. Article photochromique selon la revendication 12, comprenant un substrat ; et une couche photochromique qui est le produit durci.

14. Article photochromique selon la revendication 12 ou la revendication 13, qui est un verre de lunettes ; un verre de lunettes de protection, une partie visière d'un pare-soleil ou un élément écran d'un casque.

15. Lunettes comprenant le verre de lunettes selon la revendication 14.
